# DEMANDE DE BREVET EUROPEEN

(11) **EP 1 586 300 A1**
(43) Date de publication de la demande: **19.10.2005**
(21) Numéro de dépôt: 05290604.7
(22) Date de dépôt: 18.03.2005
(51) Int. Cl.: A61K 7/11

(54) **Dispositif aérosol contenant un agent propulseur et une composition coiffante comprenant, dans un milieu majoritairement aqueux, un polymère pseudo-bloc et un polymère fixant additionnel; procédés et utilisations**

(30) Priorité: 25.03.2004 FR 0403088
(71) Demandeur: L'OREAL, 75008 Paris (FR)
(72) Inventeur: Benabdillah, Katarina, 92110 Clichy (FR); Gawtrey, Jonathan, 92100 Boulogne (FR)
(74) Mandataire: Bulle, Françoise

(57) **Abrégé**

La présente demande concerne un dispositif aérosol contenant un agent propulseur et une composition cosmétique coiffante comprenant, dans un milieu cosmétiquement acceptable contenant majoritairement de l'eau, au moins un polymère pseudo-bloc et au moins un polymère fixant additionnel.

La présente demande concerne encore un procédé de traitement cosmétique pour la mise en forme ou le maintien de la coiffure comprenant la vaporisation sur ladite coiffure du contenu du dispositif aérosol selon la présente demande et aussi les utilisations du contenu des dispositifs aérosols pour obtenir une fixation des cheveux persistante dans le temps et/ou de bonne tenue à l'humidité et/ou de bon niveau cosmétique.

## Description

La présente invention concerne un dispositif aérosol contenant un agent propulseur et une composition cosmétique coiffante comprenant, dans un milieu cosmétiquement acceptable contenant majoritairement de l'eau, au moins un polymère pseudo-bloc et au moins un polymère fixant additionnel.

Les compositions cosmétiques pour la mise en forme et/ou le maintien de la coiffure les plus répandues sur le marché de la cosmétique sont des compositions à pulvériser essentiellement constituées d'une solution le plus souvent alcoolique et d'un ou plusieurs composants, appelés composants fixants, qui sont généralement des résines polymères statistiques dont la fonction est de former des soudures entre les cheveux. Ces composants fixants sont souvent formulés en mélange avec divers adjuvants cosmétiques. Cette composition est généralement conditionnée soit dans un récipient aérosol approprié mis sous pression à l'aide d'un agent propulseur, soit dans un flacon pompe.

Les systèmes aérosols destinés à fixer les chevelures contiennent d'une part une phase liquide (ou jus) et d'autre part un agent propulseur. La phase liquide contient les composants fixants et un solvant approprié.

Une fois appliquée sur les cheveux la phase liquide sèche, permettant la formation de soudures nécessaires à la fixation de la chevelure par les composants fixants. Les soudures doivent être suffisamment rigides pour assurer le maintien des cheveux et ce avec une persistance des effets suffisant y compris à l'humidité. Cependant, elles doivent également être suffisamment fragiles pour que l'utilisateur puisse, en peignant ou brossant la chevelure, les détruire sans heurter le cuir chevelu ni endommager les cheveux. Les compositions doivent aussi être stables dans le temps. On recherche également un bon effet cosmétique sur les cheveux en particulier au niveau de la douceur et du démêlage.

On cherche par ailleurs à diminuer la quantité de solvants volatils présents dans ces compositions pour des raisons environnementales. Le remplacement total ou partiel des solvants volatils tels que l'alcool par de l'eau se traduit généralement par une diminution spectaculaire des propriétés de fixation, de persistance de l'effet coiffant dans le temps et des propriétés cosmétiques.

De manière surprenante et avantageuse, la Demanderesse a découvert qu'une combinaison d'au moins un polymère pseudo-bloc et d'au moins un polymère fixant permet l'obtention de compositions coiffantes majoritairement aqueuses qui, introduites dans un dispositif aérosol, permettent d'obtenir une bonne fixation et un bon maintien des cheveux, c'est-à-dire un effet coiffant qui persiste tout au long de la journée, voire plusieurs jours, avec une bonne tenue à l'humidité et qui s'élimine facilement au shampooing. Ces compositions permettent également de conférer de bonnes propriétés cosmétiques aux cheveux, telles que la douceur ou le démêlage.

De plus, leur base majoritairement aqueuse rend ces compositions selon l'invention très avantageuses sur le plan écologique.

La présente invention a donc pour objet un dispositif aérosol contenant un agent propulseur et une composition cosmétique coiffante comprenant, dans un milieu cosmétiquement acceptable contenant majoritairement de l'eau, au moins un polymère pseudo-bloc et au moins un polymère fixant additionnel.

Un autre objet de l'invention consiste en un procédé de traitement cosmétique pour la mise en forme ou le maintien de la coiffure comprenant la vaporisation sur ladite coiffure du contenu d'un dispositif aérosol selon la présente demande.

Un autre objet de l'invention concerne les utilisations des dispositifs aérosols selon la présente demande pour obtenir, après vaporisation de leur contenu sur la chevelure une fixation des cheveux persistante dans le temps et/ou de bonne tenue à l'humidité et/ou de bon niveau cosmétique.

Un autre objet de l'invention concerne les utilisations des dispositifs aérosols selon la présente demande pour obtenir une mousse de coiffage.

D'autres objets, caractéristiques, aspects et avantages de l'invention apparaîtront encore plus clairement à la lecture de la description et des exemples qui suivent.

Par "composition coiffante" au sens de la présente demande, on entend une composition pour la mise en forme ou le maintien de la coiffure.

Par "milieu contenant majoritairement de l'eau" au sens de la présente demande, on entend un milieu contenant strictement plus de 50%, de préférence plus de 70%, et de manière encore plus préférée plus de 85% en poids d'eau par rapport au poids total de la composition coiffante. De préférence, ce milieu contiendra moins de 99,9% en poids d'eau par rapport au poids total de la composition coiffante.

Le milieu cosmétiquement acceptable est un milieu contenant majoritairement de l'eau et éventuellement un ou plusieurs solvants organiques.

Par "solvant organique", on entend au sens de la présente invention un composé organique de poids moléculaire inférieur à 500 liquide à la température de 25°C et à la pression atmosphérique. De préférence, le composé organique est polaire.

De préférence, ce solvant organique est un alcool. Celui-ci est notamment choisi parmi les alcools inférieurs en C₁-C₄ comme l'éthanol, l'isopropanol, le tertio-butanol, le n-butanol ; les polyols comme le propylèneglycol, les éthers de polyols et leurs mélanges, l'alcool particulièrement préféré étant l'éthanol.

Les dispositifs aérosols selon la présente demande contiennent un agent propulseur. Tous les agents propulseurs appropriés peuvent être utilisés. En particulier, les dispositifs aérosols peuvent être monocompartimentaux, des agents propulseurs sélectionnés de préférence parmi les gaz liquéfiés ou comprimés tels que l'azote, le diméthyl éther, les hydroxycarbures sont alors utilisés. Ils peuvent être aussi bicompartimentaux par exemple du type aérosol à poche avec propulsion à l'air ou avec un gaz comprimé.

La concentration en agent(s) propulseur(s) dans le dispositif représente de préférence de 6 à 70 %, plus préférentiellement de 6 à 50 %, et encore plus préférentiellement de 30 à 45 % du poids total de matières contenues dans ledit dispositif. La concentration en composition coiffante dans le dispositif aérosol représente de préférence de 30 à 94 %, plus préférentiellement de 50 à 94 %, et encore plus préférentiellement de 55 à 70 % du poids total de matières contenues dans ledit dispositif

### Polymère pseudo-bloc

Le polymère pseudo-bloc utilisé dans les compositions selon la présente invention est un polymère séquencé, comprenant au moins une première séquence et au moins une deuxième séquence incompatibles l'une avec l'autre et ayant des températures de transition vitreuse (Tg) différentes, lesdites première et deuxième séquences étant reliées entre elles par un segment intermédiaire comprenant au moins un monomère constitutif de la première séquence et au moins un monomère constitutif de la deuxième séquence et ledit polymère ayant un indice de polydispersité I supérieur à 2.

Par "au moins" une séquence, on entend une ou plusieurs séquences.

Par "séquences incompatibles l'une avec l'autre", on entend que le mélange formé du polymère correspondant à la première séquence et du polymère correspondant à la deuxième séquence, n'est pas miscible dans le solvant de polymérisation majoritaire en poids du polymère séquencé, à température ambiante (25°C) et pression atmosphérique (*10*^{*5*} Pa), pour une teneur du mélange de polymères supérieure ou égale à 5 % en poids, par rapport au poids total du mélange (polymères et solvant), étant entendu que :
i) lesdits polymères sont présents dans le mélange en une teneur telle que le rapport pondéral respectif va de 10/90 à 90/10, et que
ii) chacun des polymères correspondant aux première et seconde séquences a une masse moléculaire moyenne (en poids ou en nombre) égale à celle du polymère séquencé +/- 15%.

Dans le cas d'un mélange de solvants de polymérisation, dans l'hypothèse de deux ou plusieurs solvants présents en proportions massiques identiques, ledit mélange de polymères est non miscible dans au moins l'un d'entre eux.

Bien entendu, dans le cas d'une polymérisation réalisée dans un solvant unique, ce dernier est le solvant majoritaire.

Le segment intermédiaire est une séquence comprenant au moins un monomère constitutif de la première séquence et au moins un monomère constitutif de la deuxième séquence du polymère, il permet de "compatibiliser" ces séquences.

Le polymère séquencé de la composition selon l'invention est avantageusement un polymère éthylénique séquencé linéaire filmogène.

Par polymère "éthylénique", on entend un polymère obtenu par polymérisation de monomères comprenant une insaturation éthylénique.

Par polymère "séquencé", on entend un polymère comprenant au moins 2 séquences distinctes, de préférence au moins 3 séquences distinctes.

Le polymère est un polymère à structure linéaire. Par opposition, un polymère à structure non linéaire est, par exemple, un polymère à structure ramifiée, en étoile, greffée, ou autre.

Par polymère "filmogène", on entend un polymère apte à former à lui seul ou en présence d'un agent auxiliaire de filmification, un film continu et adhérent sur un support, notamment sur les matières kératiniques.

De façon préférentielle, le polymère pseudo-bloc utilisé selon l'invention ne comprend pas d'atomes de silicium dans son squelette. Par "squelette", on entend la chaîne principale du polymère, par opposition aux chaînes latérales pendantes.

De préférence, le polymère pseudo-bloc utilisé selon l'invention n'est pas hydrosoluble, c'est-à-dire que le polymère n'est pas soluble dans l'eau ou dans un mélange d'eau et de monoalcools inférieurs linéaires ou ramifiés ayant de 2 à 5 atomes de carbone comme l'éthanol, l'isopropanol ou le n-propanol, sans modification de pH, à une teneur en matière active d'au moins 1% en poids, à température ambiante (25°C).

De préférence, le polymère pseudo-bloc utilisé selon l'invention n'est pas un élastomère.

Par "polymère non élastomère", on entend un polymère qui, lorsqu'il est soumis à une contrainte visant à l'étirer (par exemple de 30% relativement à sa longueur initiale), ne revient pas à une longueur sensiblement identique à sa longueur initiale lorsque cesse la contrainte.

De manière plus spécifique, par "polymère non élastomére" on désigne un polymère ayant une recouvrance instantanée Rᵢ < à 50% et une recouvrance retardée R₂ₕ < 70% après avoir subi un allongement de 30%. De préférence, Rᵢ est < à 30 %, et R₂ₕ < 50%.

Plus précisément, le caractère non élastomérique du polymère pseudo-bloc utilisé est déterminé selon le protocole suivant :

On prépare un film de polymère par coulage d'une solution du polymère dans une matrice téflonnée puis séchage pendant 7 jours dans une ambiance contrôlée à 23±5°C et 50±10 % d'humidité relative.

On obtient alors un film d'environ 100 µm d'épaisseur dans lequel sont découpées des éprouvettes rectangulaires (par exemple à l'emporte-pièce) d'une largeur de 15 mm et d'une longueur de 80 mm.

On impose à cet échantillon une sollicitation de traction à l'aide d'un appareil commercialisé sous la référence Zwick, dans les mêmes conditions de température et d'humidité que pour le séchage.

Les éprouvettes sont étirées à une vitesse de 50 mm/min et la distance entre les mors est de 50 mm, ce qui correspond à la longueur initiale (I₀) de l'éprouvette.

On détermine la recouvrance instantanée Ri de la manière suivante :
- on étire l'éprouvette de 30% (εₘₐₓ) c'est-à-dire environ 0,3 fois sa longueur initiale (I₀)
- on relâche la contrainte en imposant une vitesse de retour égale à la vitesse de traction, soit 50 mm/min et on mesure l'allongement résiduel de l'éprouvette en pourcentage, après retour à contrainte charge nulle (εᵢ).

La recouvrance instantanée en % (Rᵢ) est donnée par la formule ci-après:${\text{R}}_{\text{i}} {\text{= (ε}}_{\text{max}} {\text{- ε}}_{\text{i}} {\text{)/ ε}}_{\text{max}} \text{) x 100}$

Pour déterminer la recouvrance retardée, on mesure après 2 heures le taux d'allongement résiduel de l'éprouvette en pourcentage (ε₂ₕ), 2 heures après retour à la contrainte charge nulle.

La recouvrance retardée en % (R₂ₕ) est donnée par la formule ci-après:${\text{R}}_{\text{2h}} {\text{= (ε}}_{\text{max}} {\text{- ε}}_{\text{2h}} {\text{)/ ε}}_{\text{max}} \text{) × 100}$

A titre purement indicatif, un polymère selon un mode de réalisation de l'invention possède de préférence une recouvrance instantanée Rᵢ de 10% et une recouvrance retardée R₂ₕ de 30%.

Le polymère pseudo-bloc utilisé dans les compositions selon l'invention comprend au moins une première séquence (ou bloc) et au moins une deuxième séquence (ou bloc) incompatibles l'une avec l'autre et ayant des températures de transition vitreuse (Tg) différentes, lesdites première et deuxième séquences étant reliées entre elles par un segment intermédiaire comprenant au moins un monomère constitutif de la première séquence et au moins un monomère constitutif de la deuxième séquence, ledit polymère ayant un indice de polydispersité 1 supérieur à 2.

On précise que dans ce qui précède et ce qui suit les termes "première" et "deuxième" séquences ne conditionnent nullement l'ordre desdites séquences (ou blocs) dans la structure du polymère.

L'indice de polydispersité I du polymère est égal au rapport de la masse moyenne en poids Mw sur la masse moyenne en nombre Mn.

On détermine les masses molaires moyennes en poids (Mw) et en nombre (Mn) par chromatographie liquide par perméation de gel (solvant THF, courbe d'étalonnage établie avec des étalons de polystyrène linéaire, détecteur réfractométrique).

La masse moyenne en poids (Mw) du polymère utilisé dans la composition selon l'invention est de préférence inférieure ou égale à 300 000, elle va par exemple de 35 000 à 200 000, et mieux de 45 000 à 150 000.

La masse moyenne en nombre (Mn) du polymère utilisé dans la composition selon l'invention est de préférence inférieure ou égale à 70 000, elle va par exemple de 10 000 à 60 000, et mieux de 12 000 à 50 000.

De préférence, l'indice de polydispersité du polymère utilisé dans la composition selon l'invention est supérieur à 2, par exemple allant de 2 à 9, de préférence supérieur ou égal à 2,5, par exemple allant de 2,5 à 8, et mieux supérieur ou égal à 2,8 et notamment, allant de 2,8 à 6.

Chaque séquence ou bloc du polymère utilisé dans les compositions selon l'invention est issue d'un type de monomère ou de plusieurs types de monomères différents.

Cela signifie que chaque séquence peut être constituée d'un homopolymère ou d'un copolymère, ce copolymère constituant la séquence pouvant être à son tour statistique ou alterné.

Avantageusement, le segment intermédiaire comprenant au moins un monomère constitutif de la première séquence et au moins un monomère constitutif de la deuxième séquence du polymère est un polymère statistique.

De préférence, la séquence intermédiaire est issue essentiellement de monomères constitutifs de la première séquence et de la deuxième séquence.

Par "essentiellement", on entend au moins à 85%, de préférence au moins à 90%, mieux à 95% et encore mieux à 100%.

Avantageusement, la séquence intermédiaire a une température de transition vitreuse Tg comprise entre les températures de transition vitreuse des première et deuxième séquences.

Selon l'invention, les première et deuxième séquences ont des températures de transition vitreuse différentes.

Les températures de transition vitreuse indiquées des première et deuxième séquences peuvent être des Tg théoriques déterminées à partir des Tg théoriques des monomères constitutifs de chacune des séquences, que l'on peut trouver dans un manuel de référence tel que le Polymer Handbook, 3^{rd} ed, 1989, John Wiley, selon la relation suivante, dite Loi de Fox :

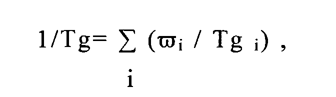

$\overline{\text{ω}}$ᵢ étant la fraction massique du monomère i dans la séquence considerée et Tgᵢ étant la température de transition vitreuse de l'homopolymère du monomère i.

Sauf indication contraire, les Tg indiquées pour les première et deuxième séquences dans la présente demande sont des Tg théoriques.

L'écart entre les températures de transition vitreuse des première et deuxième séquences est généralement supérieur à 10°C, de préférence supérieur à 20°C, et mieux supérieur à 30°C.

En particulier, la première séquence peut être choisie parmi :
- a) une séquence ayant une Tg supérieure ou égale à 40°C,
- b) une séquence ayant une Tg inférieure ou égale à 20°C,
- c) une séquence ayant une Tg comprise entre 20 et 40°C,
   et la deuxième séquence choisie dans une catégorie a), b) ou c) différente de la première séquence.

### a) Séquence ayant une Tg supérieure ou égale à 40°C

La séquence ayant une Tg supérieure ou égale à 40°C a par exemple une Tg allant de 40 à 150°C, de préférence supérieure ou égale à 50°C, allant par exemple de 50°C à 120 °C, notamment allant de 50 °C à 100 °C, et mieux supérieure ou égale à 60°C, allant par exemple de 60°C à 120°C.

La séquence ayant une Tg supérieure ou égale à 40°C peut être un homopolymère ou un copolymère.

Dans le cas où cette séquence est un homopolymère, elle est issue de monomères, qui sont tels que les homopolymères préparés à partir de ces monomères ont des températures de transition vitreuse supérieures ou égales à 40°C. Cette première séquence peut être un homopolymère, constitué par un seul type de monomère (dont la Tg de l'homopolymère correspondant est supérieure ou égale à 40°C).

Dans le cas où la première séquence est un copolymère, elle peut être issue en totalité ou en partie de un ou de plusieurs monomères, dont la nature et la concentration sont choisies de façon que la Tg du copolymère résultant soit supérieure ou égale à 40°C. Le copolymère peut par exemple comprendre :
- des monomères qui sont tels que les homopolymères préparés à partir de ces monomères ont des Tg supérieures ou égales à 40°C, par exemple une Tg allant de 40 à 150 °C, de préférence supérieure ou égale à 50°C, allant par exemple de 50°C à 120°C, et mieux supérieure
ou égale à 60°C, allant par exemple de 60°C à 120°C, et
- des monomères qui sont tels que les homopolymères préparés à partir de ces monomères ont des Tg inférieures à 40°C, choisis parmi les monomères ayant une Tg comprise entre 20 à 40°C et les monomères ayant une Tg inférieure ou égale à 20°C, par exemple une Tg allant de -100 à 20°C, de préférence inférieure à 15°C, notamment allant de - 80°C à 15°C et mieux inférieur à 10°C, par exemple allant de -50°C à 0°C à, tels que décrits plus loin, .

Les monomères dont les homopolymères ont une température de transition vitreuse supérieure ou égale à 40°C sont, de préférence, choisis parmi les monomères suivants, appelés aussi monomères principaux :
- les méthacrylates de formule CH₂ = C(CH₃)-COOR₁
   dans laquelle R₁ représente un groupe alkyle non substitué, linéaire ou ramifié, contenant de 1 à 4 atomes de carbone, tel qu'un groupe méthyle, éthyle, propyle ou isobutyle ou R₁ représente un groupe cycloalkyle C₄ à C₁₂,
- les acrylates de formule CH₂ = CH-COOR₂
   dans laquelle R₂ représente un groupe cycloalkyle en C₄ à C₁₂ tel que un groupe isobornyle ou un groupe tertio butyle,
- les (méth)acrylamides de formule : 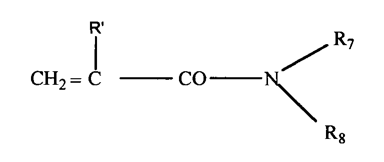 où R₇ et R₈ identiques ou différents représentent chacun un atome d'hydrogène ou un groupe alkyle en C₁ à C₁₂ linéaire ou ramifié, tel qu'un groupe n-butyle, t-butyle, isopropyle, isohexyle, isooctyle, ou isononyle ; ou R₇ représente H et R₈ représente un groupement 1,1-diméthyl-3-oxobutyl,
   et R' désigne H ou méthyle. Comme exemple de monomères, on peut citer le N-butylacrylamide, le N-t-butylacrylamide, le N-isopropylacrylamide, le N,N-diméthylacrylamide et le N,N-dibutylacrylamide ,
- et leurs mélanges.

Des monomères principaux particulièrement préférés sont le méthacrylate de méthyle, le (méth)acrylate d'isobutyle, le (méth)acrylate d'isobornyle et leurs mélanges.

### b) Séquence ayant une Tg inférieure ou égale à 20°C

La séquence ayant une Tg inférieure ou égale à 20°C a par exemple une Tg allant de -100 à 20°C, de préférence inférieure ou égale à 15°C, notamment allant de -80°C à 15°C et mieux inférieure ou égale à 10°C, par exemple allant de - 100 °C à 0°C, notamment allant de -50°C à 0°C.

La séquence ayant une Tg inférieure ou égale à 20°C peut être un homopolymère ou un copolymère.

Dans le cas où cette séquence est un homopolymère, elle est issue de monomères, qui sont tels que les homopolymères préparés à partir de ces monomères ont des températures de transition vitreuse inférieures ou égales à 20°C. Cette deuxième séquence peut être un homopolymère, constitué par un seul type de monomère (dont la Tg de l'homopolymère correspondant est inférieure ou égale à 20°C).

Dans le cas où la séquence ayant une Tg inférieure ou égale à 20°C est un copolymère, elle peut être issue en totalité ou en partie de un ou de plusieurs monomères, dont la nature et la concentration sont choisis de façon que la Tg du copolymère résultant soit inférieure ou égale à 20°C.

Elle peut par exemple comprendre
- un ou plusieurs monomères dont l'homopolymère correspondant a une Tg inférieure ou égale à 20°C, par exemple une Tg allant de -100°C à 20 °C, de préférence inférieure à 15°C, notamment allant de - 80°C à 15°C et mieux inférieur à 10°C, par exemple allant de -50°C à 0°C, et
- un ou plusieurs monomères dont l'homopolymère correspondant a une Tg supérieure à 20°C, tels que les monomères ayant une Tg supérieure ou égale à 40°C, par exemple une Tg allant de 40 à 150 °C, de préférence supérieure ou égale à 50°C, allant par exemple de 50°C à 120°C, et mieux supérieure ou égale à 60°C, allant par exemple de 60°C à 120°C et /ou les monomère ayant une Tg comprise entre 20 et 40°C, tels que décrits plus haut.

De préférence, la séquence ayant une Tg inférieure ou égale à 20°C est un homopolymère.

Les monomères dont l'homopolymère a une Tg inférieure ou égale à 20°C sont, de préférence, choisis parmi les monomères suivants, ou monomère principaux :
- les acrylates de formule CH₂ = CHCOOR₃,
   R₃ représentant un groupe alkyle non substitué en C₁ à C₁₂, linéaire ou ramifié, à l'exception du groupe tertiobutyle, dans lequel se trouve(nt) éventuellement intercalé(s) un ou plusieurs hétéroatomes choisis parmi O, N, S,
- les méthacrylates de formule CH₂ = C(CH₃)-COOR₄,
   R₄ représentant un groupe alkyle non substitué en C₆ à C₁₂ linéaire ou ramifié, dans lequel se trouve(nt) éventuellement intercalé(s) un ou plusieurs hétéroatomes choisis parmi 0, N et S;
- les esters de vinyle de formule R₅-CO-O-CH = CH₂
   où R₅ représente un groupe alkyle en C₄ à C₁₂ linéaire ou ramifié ;
   - les éthers d'alcool vinylique et d'alcool en C₄ à C₁₂,
   - les N-alkyl en C₄ à C₁₂ acrylamides, tels que le N-octylacrylamide,
   - et leurs mélanges.

Les monomères principaux particulièrement préférés pour la séquence ayant une Tg inférieure ou égale à 20°C sont les acrylates d'alkyles dont la chaîne alkyle comprend de 1 à 10 atomes de carbone, à l'exception du groupe tertiobutyle, tels que l'acrylate de méthyle, l'acrylate d'isobutyle, l'acrylate d'éthyl-2 hexyle et leurs mélanges.

### c) Séquence ayant une Tg comprise entre 20 et 40°C

La séquence qui a une Tg comprise entre 20 et 40°C peut être un homopolymère ou un copolymère.

Dans le cas où cette séquence est un homopolymère, elle est issue de monomères (ou monomère principaux), qui sont tel(s) que les homopolymères préparés à partir de ces monomères ont des températures de transition vitreuse comprises entre 20 et 40°C. Cette première séquence peut être un homopolymère, constitué par un seul type de monomère (dont la Tg de l'homopolymère correspondant va de 20°C à 40°C).

Les monomères dont l'homopolymère a une température de transition vitreuse comprise entre 20 et 40°C sont, de préférence, choisis parmi le méthacrylate de n-butyle, l'acrylate de cyclodécyle, l'acrylate de néopentyle, l'isodécylacrylamide et leurs mélanges.

Dans le cas où la séquence ayant une Tg comprise entre 20 et 40°C est un copolymère, elle est issue en totalité ou en partie de un ou de plusieurs monomères (ou monomère principaux), dont la nature et la concentration sont choisis de telle sorte que la Tg du copolymère résultant soit comprise entre 20 et 40°C.

Avantageusement, la séquence ayant une Tg comprise entre 20 et 40°C est un copolymère issue en totalité ou en partie :
- de monomères principaux dont l'homopolymère correspondant a une Tg supérieure ou égale à 40°C, par exemple une Tg allant de 40°C à 150°C, de préférence supérieure ou égale à 50°C, allant par exemple de 50 à 120°C, et mieux supérieure ou égale à 60°C, allant par exemple de 60°C à 120°C, tels que décrits plus haut, et
- de monomères principaux dont l'homopolymère correspondant a une Tg inférieure ou égale à 20°C, par exemple une Tg allant de -100 à 20°C, de préférence inférieure ou égale à 15°C, notamment allant de -80°C à 15°C et mieux inférieure ou égale à 10°C, par exemple allant de -50°C à 0°C, tels que décrits plus haut,
lesdits monomères étant choisis de telle sorte que la Tg du copolymère formant la première séquence est comprise entre 20 et 40°C.

De tels monomères principaux sont par exemple choisis parmi le méthacrylate de méthyle, l'acrylate et le méthacrylate d'isobornyle, l'acrylate de butyle, l'acrylate d'éthyl-2 hexyle et leurs mélanges.

De préférence, la proportion de la deuxième séquence ayant une Tg inférieure ou égale à 20°C va de 10 à 85% en poids du polymère, mieux de 20 à 70% et encore mieux de 20 à 50%.

Chacune des séquences peut néanmoins contenir en proportion minoritaire au moins un monomère constitutif de l'autre séquence.

Ainsi la première séquence peut contenir au moins un monomère constitutif de la deuxième séquence et inversement.

Chacune des première et/ou deuxième séquences, peu(ven)t comprendre, outre les monomères indiqués ci-dessus, un ou plusieurs autres monomères appelés monomères additionnels, différents des monomères principaux cités précédemment.

La nature et la quantité de ce ou ces monomères additionnels sont choisies de manière à ce que la séquence dans laquelle ils se trouvent ait la température de transition vitreuse désirée.

Ce monomère additionnel est par exemple choisi parmi :
a) les monomères hydrophiles tels que :
   - les monomères à insaturation(s) éthylénique(s) comprenant au moins une fonction acide carboxylique ou sulfonique comme par exemple :
      l'acide acrylique, l'acide méthacrylique, l'acide crotonique, l'anhydride maléique, l'acide itaconique, l'acide fumarique, l'acide maléique, l'acide acrylamidopropanesulfonique, l'acide vinylbenzoïque, l'acide vinylphosphorique et les sels de ceux-ci,
   - les monomères à insaturation(s) éthylénique(s) comprenant au moins une fonction amine tertiaire comme la 2-vinylpyridine, la 4-vinylpyridine, le méthacrylate de diméthylaminoéthyle, le méthacrylate de diéthylaminoéthyle, le diméthylaminopropyl méthacrylamide et les sels de ceux-ci,
   - les méthacrylates de formule CH₂ = C(CH₃)-COOR₆
      dans laquelle R₆ représente un groupe alkyle linéaire ou ramifié, contenant de 1 à 4 atomes de carbone, tel qu'un groupe méthyle, éthyle, propyle ou isobutyle, ledit groupe alkyle étant substitué par un ou plusieurs substituants choisis parmi les groupes hydroxyle (comme le méthacrylate de 2-hydroxypropyle, le méthacrylate de 2-hydroxyéthyle) et les atomes d'halogène (Cl, Br, I, F), tel que le méthacrylate de trifluoroéthyle,
   - les méthacrylates de formule CH₂ = C(CH₃)-COOR₉,
      R₉ représentant un groupe alkyle en C₆ à C₁₂ linéaire ou ramifié, dans lequel se trouve(nt) éventuellement intercalé(s) un ou plusieurs hétéroatomes choisis parmi 0, N et S, ledit groupe alkyle étant substitué par un ou plusieurs substituants choisis parmi les groupes hydroxyle et les atomes d'halogènes (Cl, Br, I, F) ;
   - les acrylates de formule CH₂ = CHCOOR₁₀,
      R₁₀ représentant un groupe alkyle en C₁ à C₁₂ linéaire ou ramifié substitué par un ou plusieurs substituants choisis parmi les groupes hydroxyle et les atomes d'halogène (Cl, Br, I et F), tel que l'acrylate de 2-hydroxypropyle et l'acrylate de 2-hydroxyéthyle, ou R₁₀ représente un alkyle en C₁ à C₁₂-O-POE (polyoxyéthylène) avec répétition du motif oxyéthylène de 5 à 30 fois, par exemple méthoxy-POE, ou R₈ représente un groupement polyoxyéthylèné comprenant de 5 à 30 motifs d'oxyde d'éthylène
b) les monomères à insaturation éthylénique comprenant un ou plusieurs atomes de silicium tels que le méthacryloxypropyl triméthoxy silane, le méthacryloxypropyl tris (triméthylsiloxy) silane,
   - et leurs mélanges.

Des monomères additionnels particulièrement préférés sont l'acide acrylique, l'acide méthacrylique, le méthacrylate de trifluoroéthyle et leurs mélanges.

Selon un mode préféré de réalisation, le polymère utilisé dans les compositions selon l'invention est un polymère non siliconé, c'est à dire un polymère exempt d'atome de silicium.

Ce ou ces monomères additionnels représente(nt) généralement une quantité inférieure ou égale à 30% en poids, par exemple de 1 à 30% en poids, de préférence de 5 à 20% en poids et, de préférence encore, de 7 à 15% en poids du poids total des première et/ou deuxième séquences.

De préférence, chacune des première et deuxième séquences comprend au moins un monomère choisi parmi les esters d'acide (méth)acrylique, et éventuellement au moins un monomère choisi parmi l'acide (méth)acrylique, et leurs mélanges.

Avantageusement, chacune des première et deuxième séquences est issue en totalité d'au moins un monomère choisi parmi l'acide acrylique, les esters d'acide (méth)acrylique, et éventuellement d'au moins un monomère choisi parmi l'acide (méth)acrylique, et leurs mélanges

De préférence, le polymère utilisé dans les compositions selon l'invention est exempt de styrène. Par "polymère exempt de styrène", on entend un polymère contenant moins de 10 % en poids, par rapport au poids total du polymère, de préférence moins de 5 % en poids, mieux moins de 2 % en poids, mieux moins de 1 % en poids, voire ne contenant pas, de monomère styrénique comme le styrène, les dérivés de styrène tels que le méthylstyrène, le chlorostyrène ou le chlorométhylstyrène.

Le polymère utilisé dans les compositions selon l'invention peut être obtenu par polymérisation radicalaire en solution selon le procédé de préparation suivant :
- une partie du solvant de polymérisation est introduite dans un réacteur adapté et chauffée jusqu'à atteindre la température adéquate pour la polymérisation (typiquement entre 60 et 120°C),
- une fois cette température atteinte, les monomères constitutifs de la première séquence sont introduits en présence d'une partie de l'initiateur de polymérisation,
- au bout d'un temps T correspondant à un taux de conversion maximum de 90%, les monomères constitutifs de la deuxième séquence et l'autre partie de l'initiateur sont introduits,
- on laisse réagir le mélange pendant un temps T' (allant de 3 à 6 h) au bout duquel le mélange est ramené à température ambiante,
- on obtient le polymère en solution dans le solvant de polymérisation.

Par "solvant de polymérisation", on entend un solvant ou un mélange de solvants. Le solvant de polymérisation peut être choisis notamment parmi l'acétate d'éthyle, l'acétate de butyle, les alcools tels que l'isopropanol, l'éthanol, les alcanes aliphatiques tels que l'isododécane et leurs mélanges. De préférence, le solvant de polymérisation est un mélange acétate de butyle et isopropanol ou l'isododécane.

### Premier mode de réalisation

Selon un premier mode de réalisation, le polymère utilisé dans les compositions selon l'invention comprend au moins une (notamment une) première séquence ayant une Tg supérieure ou égale à 40°C, telle que décrite plus haut au paragraphe a) et au moins une (notamment une) deuxième séquence ayant une Tg inférieure ou égale à 20°C, telle que décrite plus haut au paragraphe b).

De préférence, la première séquence ayant une Tg supérieure ou égale à 40°C est un copolymère issu de monomères qui sont tels que l'homopolymère préparé à partir de ces monomères a une température de transition vitreuse supérieure ou égale à 40°C, tels que les monomères décrits plus haut.

Avantageusement, la deuxième séquence ayant une Tg inférieure ou égale à 20°C est un homopolymère issu de monomères qui sont tel(s) que l'homopolymère préparé à partir de ces monomères a une température de transition vitreuse inférieure ou égale à 20°C, tels que les monomères décrits plus haut.

De préférence, la proportion de la séquence ayant une Tg supérieure ou égale à 40°C va de 20 à 90% en poids du polymère, mieux de 30 à 80% et encore mieux de 50 à 70%.

De préférence, la proportion de la séquence ayant une Tg inférieure ou égale à 20°C va de 5 à 75% en poids du polymère, de préférence de 15 à 50% et mieux de 25 à 45%.

Ainsi, selon une première variante, le polymère utilisé dans les compositions selon l'invention peut comprendre :
- une première séquence de Tg supérieure ou égale à 40°C, par exemple ayant une Tg allant de 70 à 110°C, qui est un copolymère méthacrylate de méthyle / acide acrylique,
- une deuxième séquence de Tg inférieure ou égale à 20°C, par exemple allant de 0 à 20°C, qui est un homopolymère d'acrylate de méthyle et
- une séquence intermédiaire qui est un copolymère méthacrylate de méthyle/acide acrylique/acrylate de méthyle.

Selon une seconde variante, le polymère utilisé dans les compositions selon l'invention peut comprendre :
- une première séquence de Tg supérieure ou égale à 40°C, par exemple allant de 70 à 100°C, qui est un copolymère méthacrylate de méthyle/acide acrylique/méthacrylate de trifluoroéthyle,
- une deuxième séquence de Tg inférieure ou égale à 20°C, par exemple allant de 0 à 20°C, qui est un homopolymère d'acrylate de méthyle et
- une séquence intermédiaire qui est un copolymère statistique méthacrylate de méthyl/acide acrylique/acrylate de méthyle/méthacrylate de trifluoroéthyle.

Selon une troisième variante, le polymère utilisé dans les compositions selon l'invention peut comprendre :
- une première séquence de Tg supérieure ou égale à 40°C, par exemple allant de 85 à 115°C, qui est un copolymère acrylate d'isobornyle/méthacrylate d'isobutyle,
- une deuxième séquence de Tg inférieure ou égale à 20°C, par exemple allant de -85 à -55°C, qui est un homopolymère d'acrylate d'éthyl-2 hexyle et
- une séquence intermédiaire qui est un copolymère statistique acrylate d'isobornyle/méthacrylate d'isobutyle/acrylate d'éthyl-2 hexyle.

Selon une quatrième variante, le polymère utilisé dans les compositions selon l'invention peut comprendre :
- une première séquence de Tg supérieure ou égale à 40°C, par exemple allant de 85 à 115°C, qui est un copolymère acrylate d'isobornyle/méthacrylate de méthyle,
- une deuxième séquence de Tg inférieure ou égale à 20°C, par exemple allant de -85 à -55°C, qui est un homopolymère d'acrylate d'éthyl-2 hexyle et
- une séquence intermédiaire qui est un copolymère statistique acrylate d'isobornyle/méthacrylate de méthyle/acrylate d'éthyl-2 hexyle.

Selon une cinquième variante, le polymère utilisé dans les compositions selon l'invention peut comprendre :
- une première séquence de Tg supérieure ou égale à 40°C, par exemple allant de 95 à 125°C, qui est un copolymère acrylate d'isobornyle / méthacrylate d'isobornyle,
- une deuxième séquence de Tg inférieure ou égale à 20°C, par exemple allant de -85 à -55°C, qui est un homopolymère d'acrylate d'éthyl-2 hexyle et
- une séquence intermédiaire qui est un copolymère statistique acrylate d'isobornyle/ méthacrylate d'isobornyle/ acrylate d'éthyl-2 hexyle.

Selon une sixième variante, le polymère utilisé dans les compositions selon l'invention peut comprendre :
- une première séquence de Tg supérieure ou égale à 40°C, par exemple allant de 85 à 115°C, qui est un copolymère méthacrylate d'isobornyle/méthacrylate d'isobutyle,
- une deuxième séquence de Tg inférieure ou égale à 20°C, par exemple allant de -35 à -5°C, qui est un homopolymère d'acrylate d'isobutyle et
- une séquence intermédiaire qui est un copolymère statistique méthacrylate d'isobornyle/méthacrylate d'isobutyle/acrylate d'isobutyle.

Selon une septième variante, le polymère utilisé dans les compositions selon l'invention peut comprendre :
- une première séquence de Tg supérieure ou égale à 40°C, par exemple allant de 95 à 125°C, qui est un copolymère acrylate d'isobornyle/méthacrylate d'isobornyle,
- une deuxième séquence de Tg inférieure ou égale à 20°C, par exemple allant de -35 à -5°C, qui est un homopolymère d'acrylate d'isobutyle et
- une séquence intermédiaire qui est un copolymère statistique acrylate d'isobornyle/méthacrylate d'isobornyle/acrylate d'isobutyle.

Selon une huitième variante, le polymère utilisé dans les compositions selon l'invention peut comprendre :
- une première séquence de Tg supérieure ou égale à 40°C, par exemple allant de 60 à 90°C, qui est un copolymère acrylate d'isobornyle/méthacrylate d'isobutyle,
- une deuxième séquence de Tg inférieure ou égale à 20°C, par exemple allant de -35 à -5°C, qui est un homopolymère d'acrylate d'isobutyle et
- une séquence intermédiaire qui est un copolymère statistique acrylate d'isobornyle/méthacrylate d'isobutyle/acrylate d'isobutyle.

Selon une neuvième variante, le polymère utilisé dans les compositions selon l'invention peut comprendre :
- une première séquence de Tg supérieure ou égale à 40°C, par exemple ayant une Tg allant de 70 à 110°C, qui est un copolymère acide acrylique/acrylate de méthyle,
- une deuxième séquence de Tg inférieure ou égale à 20°C, par exemple allant de 0 à 20°C, qui est un homopolymère d'acrylate de méthyle et
- une séquence intermédiaire qui est un copolymère acide acrylique/acrylate de méthyle/polyacrylate de méthyle.

### Second mode de réalisation

Selon un second mode de réalisation, le polymère utilisé dans les compositions selon l'invention comprend au moins une (notamment une) première séquence ayant une température de transition vitreuse (Tg) comprise entre 20 et 40°C, conforme aux séquences décrites au paragraphe c) et au moins une (notamment une) deuxième séquence ayant une température de transition vitreuse inférieure ou égale à 20°C, telle que décrite plus haut au paragraphe b) ou une température de transition vitreuse supérieure ou égale à 40°C, telle que décrite au paragraphe a) ci-dessus.

De préférence, la proportion de la première séquence ayant une Tg comprise entre 20 et 40°C va de 10 à 85% en poids du polymère, mieux de 30 à 80% et encore mieux de 50 à 70%.

Lorsque la deuxième séquence est une séquence ayant une Tg supérieure ou égale à 40°C, elle est de préférence présente en une proportion allant de 10 à 85% en poids du polymère, mieux de 20 à 70% et encore mieux de 30 à 70%.

Lorsque la deuxième séquence est une séquence ayant une Tg inférieure ou égale à 20°C, elle est de préférence présente en une proportion allant de 10 à 85% en poids du polymère, mieux de 20 à 70% et encore mieux de 20 à 50%.

De préférence, la première séquence ayant une Tg comprise entre 20 et 40°C est un copolymère issu de monomères qui sont tel(s) que l'homopolymère correspondant a une Tg supérieure ou égale à 40°C et de monomères qui sont tel(s) que l'homopolymère correspondant a une Tg inférieure ou égale à 20°C.

Avantageusement, la deuxième séquence ayant une Tg inférieure ou égale à 20°C ou ayant une Tg supérieure ou égale à 40°C est un homopolymère.

Ainsi, selon première variante de ce second mode de réalisation, le polymère utilisé dans les compositions selon l'invention peut comprendre :
- une première séquence de Tg comprise entre 20 et 40°C, par exemple ayant une Tg de 25 à 39°C, qui est un copolymère comprenant au moins un monomère acrylate de méthyle, au moins un monomère méthacrylate de méthyle et au moins un monomère acide acrylique,
- une deuxième séquence de Tg supérieure ou égale à 40°C, par exemple allant de 85 à 125°C, qui est un homopolymère composé de monomères méthacrylate de méthyle et
- une séquence intermédiaire comprenant au moins un monomère acrylate de méthyle, méthacrylate de méthyle et
- une séquence intermédiaire comprenant au méthacrylate de méthyle, au moins un monomère acide acrylique et au moins un monomère acrylate de méthyle.

Selon seconde variante de ce second mode de réalisation, le polymère utilisé dans les compositions selon l'invention peut comprendre :
- une première séquence de Tg comprise entre 20 et 40°C, par exemple ayant une Tg de 21 à 39°C, qui est un copolymère comprenant acrylate d'isobornyle/méthacrylate d'isobutyle/acrylate d'éthyl-2 hexyle,
- une deuxième séquence de Tg inférieure ou égale à 20°C, par exemple allant de -65 à -35°C, qui est un homopolymère de méthacrylate de méthyle et
- une séquence intermédiaire qui est un copolymère statistique acrylate d'isobornyle /méthacrylate d'isobutyle/acrylate d'éthyl-2 hexyle.

Selon une troisième variante de ce second mode de réalisation, le polymère utilisé dans les compositions selon l'invention peut comprendre :
- une première séquence de Tg comprise entre 20 et 40°C, par exemple ayant une Tg de 21 à 39°C, qui est un copolymère acrylate d'isobornyle/acrylate de méthyle/acide acrylique,
- une deuxième séquence de Tg supérieure ou égale à 40°C, par exemple allant de 85 à 115°C, qui est un homopolymère d'acrylate d'isobornyle et
- une séquence intermédiaire qui est un copolymère statistique acrylate d'isobornyle / acrylate de méthyle/acide acrylique.

La composition cosmétique coiffante selon l'invention comprend de préférence le ou les polymère(s) pseudo-bloc(s) en une quantité allant de 0,1 à 60% en poids, de préférence de 0,5 à 50% en poids et de manière encore plus préférée de 1 à 40 % en poids par rapport au poids total de la composition.

### Polymère fixant additionnel

La composition pour dispositif aérosol comprend en outre un deuxième polymère fixant différent du polymère pseudo-bloc utilisé, ce deuxième polymère fixant est appelé polymère fixant additionnel dans la suite de la description.

Tous les polymères fixants anioniques, cationiques, amphotères, non ioniques et leurs mélanges utilisés dans la technique peuvent être utilisés dans les compositions selon la présente demande.

Les polymères fixants additionnel peuvent être solubles dans le milieu cosmétiquement acceptable ou insolubles dans ce même milieu et utilisés dans ce cas sous forme de dispersions de particules solides ou liquides de polymère (latex ou pseudolatex).

Les polymères fixants anioniques généralement utilisés sont des polymères comportant des groupements dérivés d'acide carboxylique, sulfonique ou phosphorique et ont une masse moléculaire moyenne en nombre comprise entre environ 500 et 5 000 000.

Les groupements carboxyliques sont apportés par des monomères mono ou diacides carboxyliques insaturés tels que ceux répondant à la formule : 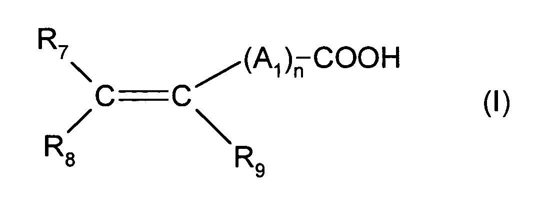 dans laquelle n est un nombre entier de 0 à 10, A, désigne un groupement méthylène, éventuellement relié à l'atome de carbone du groupement insaturé ou au groupement méthylène voisin lorsque n est supérieur à 1, par l'intermédiaire d'un hétéroatome tel qu'oxygène ou soufre, R₇ désigne un atome d'hydrogène, un groupement phényle ou benzyle, R₈ désigne un atome d'hydrogène, un groupement alkyle inférieur ou carboxyle, R₉ désigne un atome d'hydrogène, un groupement alkyle inférieur, un groupement -CH₂-COOH, phényle ou benzyle.

Dans la formule précitée, un groupement alkyle inférieur désigne de préférence un groupement ayant 1 à 4 atomes de carbone et en particulier, les groupements méthyle et éthyle.

Les polymères fixants anioniques à groupements carboxyliques préférés selon l'invention sont :
A) Les homo- ou copolymères d'acide acrylique ou méthacrylique ou leurs sels et en particulier les produits vendus sous les dénominations VERSICOL® E ou K par la société ALLIED COLLOID et ULTRAHOLD® par la société BASF, les copolymères d'acide acrylique et d'acrylamide vendus sous la forme de leurs sels de sodium sous les dénominations RETEN 421, 423 ou 425 par la Société HERCULES, les sels de sodium des acides polyhydroxycarboxyliques ;
B) Les copolymères d'acide acrylique ou méthacrylique avec un monomère monoéthylénique tel que l'éthylène, le styrène, les esters vinyliques, les esters d'acide acrylique ou méthacrylique, éventuellement greffés sur un polyalkylène-glycol tel que le polyéthylène-glycol, et éventuellement réticulés. De tels polymères sont décrits en particulier dans le brevet français n° 1 222 944 et la demande allemande n° 2 330 956, les copolymères de ce type comportant dans leur chaîne un motif acrylamide éventuellement N-alkylé et/ou hydroxyalkylé tels que décrits notamment dans les demandes de brevet luxembourgeois n^{os} 75370 et 75371 ou proposés sous la dénomination QUADRAMER par la société AMERICAN CYANAMID. On peut également citer les copolymères d'acide acrylique et de méthacrylate d'alkyle en C₁-C₄ et les terpolymères de vinylpyrrolidone, d'acide acrylique et de méthacrylate d'alkyle en C,-C₂₀, par exemple, de lauryle, tels que celui commercialisé par la société ISP sous la dénomination ACRYLIDONE® LM et les terpolymères acide méthacrylique/acrylate d'éthyle/acrylate de tertiobutyle tels que le produit commercialisé sous la dénomination LUVIMER® 100 P par la société BASF ;
   On peut aussi citer les copolymères acide méthacrylique/ acide acrylique/acrylate d'éthyle/méthacrylate de méthyle en dispersion aqueuse, commercialisé sous la dénomination AMERHOLD® DR 25 par la société AMERCHOL ;
C) Les copolymères d'acide crotonique, tels que ceux comportant dans leur chaîne des motifs acétate ou propionate de vinyle, et éventuellement d'autres monomères tels que les esters allylique ou méthallylique, éther vinylique ou ester vinylique d'un acide carboxylique saturé, linéaire ou ramifié, à longue chaîne hydrocarbonée, comme ceux comportant au moins 5 atomes de carbone, ces polymères pouvant éventuellement être greffés ou réticulés, ou encore un autre monomère ester vinylique, allylique ou méthallylique d'un acide carboxylique α- ou β-cyclique. De tels polymères sont décrits entre autres dans les brevets français n^{os} 1 222 944, 1 580 545, 2 265 782, 2 265 781, 1 564 110 et 2 439 798. Des produits commerciaux entrant dans cette classe sont les résines 28-29-30, 26-13-14 et 28-13-10 commercialisées par la société National Starch ;
D) Les copolymères d'acides ou d'anhydrides carboxyliques monoinsaturés en C₄-C₈ choisis parmi :
   - les copolymères comprenant (i) un ou plusieurs acides ou anhydrides maléique, fumarique, itaconique et (ii) au moins un monomère choisi parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées. De tels polymères sont décrits en particulier dans les brevets US n^{os} 2 047 398, 2 723 248, 2 102 113, le brevet GB n° 839 805. Des produits commerciaux sont notamment ceux vendus sous les dénominations GANTREZ® AN ou ES par la société ISP ;
   - les copolymères comprenant (i) un ou plusieurs motifs anhydrides maléique, citraconique, itaconique et (ii) un ou plusieurs monomères choisis parmi les esters allyliques ou méthallyliques comportant éventuellement un ou plusieurs groupements acrylamide, méthacrylamide, α-oléfine, esters acryliques ou méthacryliques, acides acrylique ou méthacrylique ou vinylpyrrolidone dans leur chaîne,
      les fonctions anhydrides de ces copolymères étant éventuellement monoestérifiées ou monoamidifiées.

   Ces polymères sont par exemple décrits dans les brevets français n^{os} 2 350 384 et 2 357 241 de la demanderesse ;
E) Les polyacrylamides comportant des groupements carboxylates.

Les homopolymères et copolymères comprenant des groupements sulfoniques sont des polymères comportant des motifs vinylsulfonique, styrène-sulfonique, naphtalène-sulfonique ou acrylamido-alkylsulfonique.

Ces polymères peuvent être notamment choisis parmi :
- les sels de l'acide polyvinylsulfonique ayant une masse moléculaire comprise entre environ 1 000 et 100 000, ainsi que les copolymères avec un comonomère insaturé tel que les acides acrylique ou méthacrylique et leurs esters, ainsi que l'acrylamide ou ses dérivés, les éthers vinyliques et la vinylpyrrolidone ;
- les sels de l'acide polystyrène-sulfonique tels que les sels de sodium vendus par exemple sous la dénomination Flexan® 500 et Flexan® 130 par National Starch. Ces composés sont décrits dans le brevet FR 2 198 719 ;
- les sels d'acides polyacrylamide-sulfoniques tels que ceux mentionnés dans le brevet US 4 128 631, et plus particulièrement l'acide polyacrylamidoéthylpropane-sulfonique vendu sous la dénomination COSMEDIA POLYMER HSP 1180 par HENKEL.
Comme autre polymère fixant anionique utilisable selon l'invention, on peut citer le polymère anionique séquencé branché vendu sous la dénomination Fixate G-100 par la société NOVEON.

Selon l'invention, les polymères fixants anioniques sont de préférence choisis parmi les copolymères d'acide acrylique tels que les terpolymères acide acrylique/acrylate d'éthyle/N-tertiobutylacrylamide vendus notamment sous la dénomination ULTRAHOLD® STRONG par la société BASF, les copolymères dérivés d'acide crotonique tels que les terpolymères acétate de vinyle/tertio-butylbenzoate de vinyle/acide crotonique et les terpolymères acide crotonique/acétate de vinyle/néododécanoate de vinyle vendus notamment sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique monoestérifié vendus, par exemple, sous la dénomination GANTREZ® ES 425 par la société ISP, les copolymères d'acide méthacrylique et de méthacrylate de méthyle vendus sous la dénomination EUDRAGIT® L par la société ROHM PHARMA, les copolymères d'acide méthacrylique et d'acrylate d'éthyle vendus sous la dénomination LUVIMER® MAEX ou MAE par la société BASF et les copolymères acétate de vinyle/acide crotonique vendus sous la dénomination LUVISET CA 66 par la société BASF et les copolymères acétate de vinyle/acide crotonique greffés par du polyéthylèneglycol vendus sous la dénomination ARISTOFLEX® A par la société BASF, le polymère vendu sous la dénomination Fixate G-100 par la société NOVEON.

Parmi les polymères fixants anioniques cités ci-dessus, on préfère plus particulièrement utiliser dans le cadre de la présente invention, les copolymères méthylvinyléther/anhydride maléique monoestérifiés vendus sous la dénomination GANTREZ® ES 425 par la société ISP, les terpolymères acide acrylique/acrylate d'éthyle/N-tertiobutylacrylamide vendus sous la dénomination ULTRAHOLD® STRONG par la société BASF, les copolymères d'acide méthacrylique et de méthacrylate de méthyle vendus sous la dénomination EUDRAGIT® L par la société ROHM PHARMA, les terpolymères acétate de vinyle/tertio-butylbenzoate de vinyle/acide crotonique et les terpolymères acide crotonique/acétate de vinyle/ néododécanoate de vinyle vendus sous la dénomination Résine 28-29-30 par la société NATIONAL STARCH, les copolymères d'acide méthacrylique et d'acrylate d'éthyle vendus sous la dénomination LUVIMER® MAEX OU MAE par la société BASF, les terpolymères vinylpyrrolidone/acide acrylique/méthacrylate de lauryle vendus sous la dénomination ACRYLIDONE® LM par la société ISP, le polymère vendu sous la dénomination Fixate G-100 par la société NOVEON.

Les polymères filmogènes fixants cationiques utilisables selon la présente invention sont de préférence choisis parmi les polymères comportant des groupements amines primaires, secondaires, tertiaires et/ou quaternaires faisant partie de la chaîne polymère ou directement reliés à celle-ci, et ayant un poids moléculaire compris entre 500 et environ 5 000 000 et de préférence entre 1 000 et 3 000 000.

Parmi ces polymères, on peut citer plus particulièrement les polymères cationiques suivants :
(1) les homopolymères ou copolymères dérivés d'esters ou d'amides acryliques ou méthacryliques et comportant au moins un des motifs de formules suivantes : 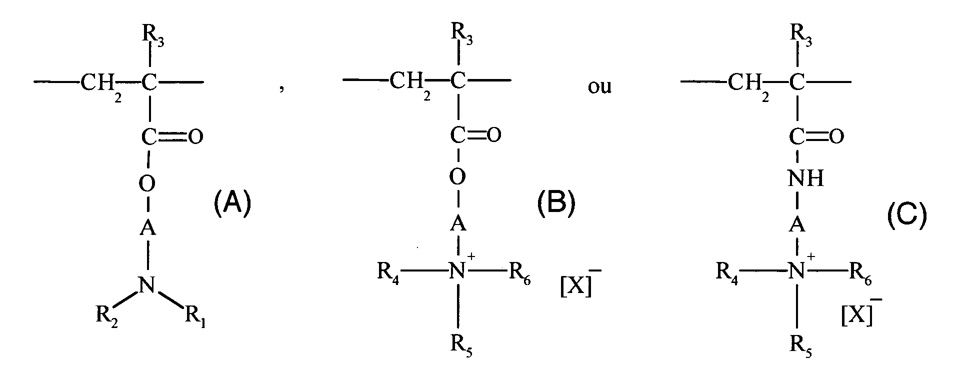 dans lesquelles:
   R₃ désigne un atome d'hydrogène ou un radical CH₃;
   A est un groupe alkyle linéaire ou ramifié comportant de 1 à 6 atomes de carbone ou un groupe hydroxyalkyle comportant de 1 à 4 atomes de carbone ;
   R₄, R₅, R₆, identiques ou différents, représentent un groupe alkyle ayant de 1 à 18 atomes de carbone ou un radical benzyle;
   R₁ et R₂, identiques ou différents, représentent chacun un atome hydrogène ou un groupe alkyle ayant de 1 à 6 atomes de carbone;
   X désigne un anion méthosulfate ou un halogénure tel que chlorure ou bromure.

   Les copolymères de la famille (1) contiennent en outre un ou plusieurs motifs dérivant de comonomères pouvant être choisis dans la famille des acrylamides, méthacrylamides, diacétones-acrylamides, acrylamides et méthacrylamides substitués sur l'azote par des groupes alkyles inférieurs (C1-C4), des groupes dérivés des acides acryliques ou méthacryliques ou leurs esters, des vinyllactames tels que la vinylpyrrolidone ou le vinylcaprolactame, d'esters vinyliques.
   Ainsi, parmi ces copolymères de la famille (1), on peut citer :
   - les copolymères d'acrylamide et de méthacrylate de diméthylaminoéthyle quaternisé au sulfate de diméthyle ou avec un halogénure de diméthyle, tels que celui vendu sous la dénomination HERCOFLOC® par la société HERCULES,
   - les copolymères d'acrylamide et de chlorure de méthacryloyloxyéthyltriméthylammonium décrit par exemple dans la demande de brevet EP-A-080976 et vendus sous la dénomination BINA QUAT P 100 par la société CIBA GEIGY,
   - le copolymère d'acrylamide et de méthosulfate de méthacryloyloxyéthyltriméthylammonium tel que celui vendu sous la dénomination RETEN par la société HERCULES,
   - les copolymères vinylpyrrolidone/acrylate ou méthacrylate de dialkylaminoalkyle quaternisés ou non, tels que les produits vendus sous la dénomination "GAFQUAT® " par la société ISP comme par exemple "GAFQUAT® 734" ou "GAFQUAT® 755" ou bien les produits dénommés "COPOLYMER® 845, 958 et 937". Ces polymères sont décrits en détail dans les brevets français 2.077.143 et 2.393.573,
   - les polymères à chaîne grasse et à motif vinylpyrrolidone, tels que les produits vendus sous la dénomination Stylèze W20 et Stylèze W10 par la société ISP,
   - les terpolymères méthacrylate de diméthylaminoéthyle/vinylcaprolactame/vinylpyrrolidone tel que le produit commercialisé sous la dénomination GAFFIX VC 713 par la société ISP, et
   - et les copolymères vinylpyrrolidone/méthacrylamide de diméthylaminopropyle quaternisés tels que les produits commercialisés sous la dénomination "GAFQUAT® HS 100" par la société ISP.
(2) les polysaccharides cationiques, de préférence à ammonium quaternaire tels que ceux décrits dans les brevets américains 3.589.578 et 4.031.307 tel que les gommes de guar contenant des groupements cationiques trialkylammonium. De tels produits sont commercialisés notamment sous les dénominations commerciales de JAGUAR C13 S, JAGUAR C 15, JAGUAR C 17 par la société MEYHALL.
(3) les copolymères quaternaires de vinylpyrrolidone et de vinylimidazole ;
(4) les chitosanes ou leurs sels; les sels utilisables sont en particulier les acétate, lactate, glutamate, gluconate ou le pyrrolidone-carboxylate de chitosane .
   Parmi ces composés, on peut citer le chitosane ayant un taux de désacétylation de 90,5% en poids vendu sous la dénomination KYTAN BRUT STANDARD par la société ABER TECHNOLOGIES, le pyrrolidone-carboxylate de chitosane commercialisé sous la dénomination KYTAMER® PC par la société AMERCHOL .
(5) les dérivés de cellulose cationiques tels que les copolymères de cellulose ou de dérivés de cellulose greffés avec un monomère hydrosoluble comportant un ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyloxyéthyl triméthylammonium, de méthacrylamidopropyl triméthylammonium, de diméthyl-diallylammonium.

Les produits commercialisés répondant à cette définition sont plus particulièrement les produits vendus sous la dénomination "CELQUAT L 200" et "CELQUAT H 100" par la Société National Starch.

Les polymères fixants amphotères utilisables conformément à l'invention peuvent être choisis parmi les polymères comportant des motifs B et C répartis statistiquement dans la chaîne polymère où B désigne un motif dérivant d'un monomère comportant au moins un atome d'azote basique et C désigne un motif dérivant d'un monomère acide comportant un ou plusieurs groupements carboxyliques ou sulfoniques, ou bien B et C peuvent désigner des groupements dérivant de monomères zwittérioniques de carboxybétaïnes ou de sulfobétaïnes ;

B et C peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un groupe hydrocarboné, ou bien B et C font partie d'une chaîne d'un polymère à motif éthylène-α,β-dicarboxylique dont l'un des groupements carboxyliques a été amené à réagir avec une polyamine comportant un ou plusieurs groupements amines primaires ou secondaires.

Les polymères fixants amphotères répondant à la définition donnée ci-dessus plus particulièrement préférés sont choisis parmi les polymères suivants :
(1) les copolymères à motifs vinyliques acides et à motifs vinyliques basiques, tels que ceux résultant de la copolymérisation d'un monomère dérivé d'un composé vinylique portant un groupement carboxylique tel que plus particulièrement l'acide acrylique, l'acide méthacrylique, l'acide maléïque, l'acide alpha-chloracrylique, et d'un monomère basique dérivé d'un composé vinylique substitué contenant au moins un atome basique, tel que plus particulièrement les méthacrylate et acrylate de dialkylaminoalkyle, les dialkylaminoalkylméthacrylamide et acrylamide. De tels composés sont décrits dans le brevet américain n° 3 836 537.
(2) les polymères comportant des motifs dérivant :
   a) d'au moins un monomère choisi parmi les acrylamides ou les méthacrylamides substitués sur l'atome d'azote par un groupe alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique, et le produit de quaternisation du méthacrylate de diméthylaminoéthyle avec le sulfate de diméthyle ou diéthyle.

   Les acrylamides ou méthacrylamides N-substitués plus particulièrement préférés selon l'invention sont les composés dont les groupes alkyles comportent de 2 à 12 atomes de carbone, et plus particulièrement le N-éthylacrylamide, le N-tertiobutylacrylamide, le N-tertiooctylacrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléïque ou fumarique.
   Les comonomères basiques préférés sont des méthacrylates d'aminoéthyle, de butylaminoéthyle, de N,N'-diméthylaminoéthyle, de N-tertio-butylaminoéthyle.
   On utilise particulièrement les copolymères dont la dénomination CTFA (4ème Ed., 1991) est Octylacrylamide/acrylates/butylaminoethyl-methacrylate copolymer, tels que les produits vendus sous la dénomination AMPHOMER® ou LOVOCRYL® 47 par la société NATIONAL STARCH.
(3) les polyaminoamides réticulés et acylés partiellement ou totalement dérivant de polyaminoamides de formule générale : 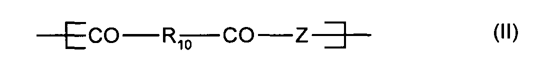 dans laquelle R₁₀ représente un groupe divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un groupe dérivant de l'addition de l'un quelconque desdits acides avec une amine bis-primaire ou bis-secondaire, et Z désigne un groupe dérivant d'une polyalkylène-polyamine bis-primaire, mono- ou bis-secondaire et de préférence représente :
   a) dans les proportions de 60 à 100 % en moles, le groupe 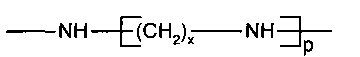 où x=2 et p=2 ou 3, ou bien x=3 et p=2
      ce groupe dérivant de la diéthylène-triamine, de la triéthylènetétraamine ou de la dipropylène-triamine ;
   b) dans les proportions de 0 à 40 % en moles, le groupe (IV) ci-dessus, dans lequel x=2 et p=1 et qui dérive de l'éthylène-diamine, ou le groupe dérivant de la pipérazine : 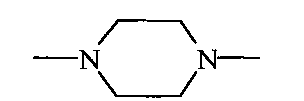
   c) dans les proportions de 0 à 20 % en moles, le groupe -NH-(CH₂)₆-NH- dérivant de l'hexaméthylènediamine, ces polyaminoamides étant réticulés par réaction d'addition d'un agent réticulant bifonctionnel choisi parmi les épihalohydrines, les diépoxydes, les dianhydrides, les dérivés bis-insaturés, au moyen de 0,025 à 0,35 mole d'agent réticulant par groupement amine du polyaminoamide, et acylés par action d'acide acrylique, d'acide chloracétique ou d'une alcane-sultone ou de leurs sels.

   Les acides carboxyliques saturés sont choisis de préférence parmi les acides ayant 6 à 10 atomes de carbone tels que les acides adipique, triméthyl-2,2,4-adipique et triméthyl-2,4,4-adipique, téréphtalique, les acides à double liaison éthylénique comme, par exemple, les acides acrylique, méthacrylique, itaconique.
   Les alcane-sultones utilisées dans l'acylation sont de préférence la propane- ou la butane-sultone, les sels des agents d'acylation sont de préférence les sels de sodium ou de potassium.
(4) les polymères comportant des motifs zwittérioniques de formule : 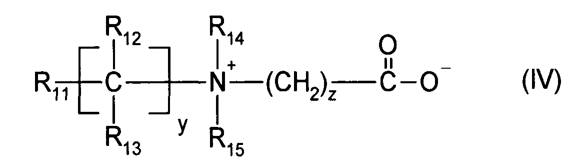 dans laquelle R₁₁ désigne un groupement insaturé polymérisable tel qu'un groupement acrylate, méthacrylate, acrylamide ou méthacrylamide, y et z représentent un nombre entier de 1 à 3, R₁₂ et R₁₃ représentent un atome d'hydrogène, un groupe méthyle, éthyle ou propyle, R₁₄ et R₁₅ représentent un atome d'hydrogène ou un groupe alkyle de telle façon que la somme des atomes de carbone dans R₁₄ et R₁₅ ne dépasse pas 10.
   Les polymères comprenant de tels motifs peuvent également comporter des motifs dérivés de monomères non zwittérioniques tels que l'acrylate ou le méthacrylate de diméthyl- ou diéthylaminoéthyle ou des acrylates ou méthacrylates d'alkyle, des acrylamides ou méthacrylamides, ou l'acétate de vinyle.
   A titre d'exemple, on peut citer les copolymères méthacrylate de méthyle/diméthyl-carboxyméthylammonio-éthylméthacrylate de méthyle, tel que le produit vendu sous la dénomination DIAFORMER Z301 par la société SANDOZ.
(5) les polymères dérivés du chitosane comportant des motifs monomères répondant aux formules suivantes : 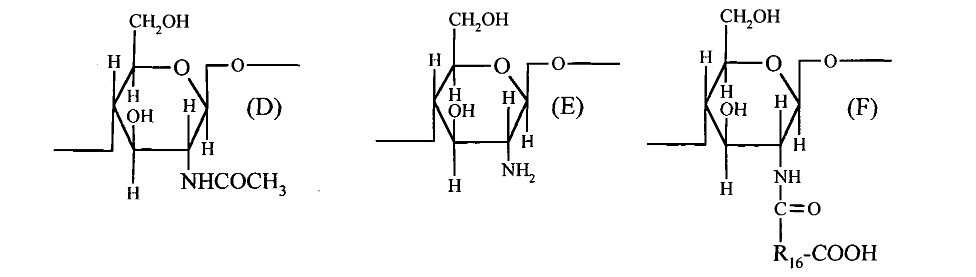 le motif (D) étant présent dans des proportions comprises entre 0 et 30%, le motif (E) dans des proportions comprises entre 5 et 50% et le motif (F) dans des proportions comprises entre 30 et 90%, étant entendu que dans ce motif (F), R₁₆ représente un groupe de formule : 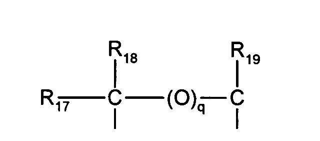 dans laquelle si q=0, R₁₇, R₁₈ et R₁₉, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des groupes R₁₇, R₁₈ et R₁₉ étant dans ce cas un atome d'hydrogène ;
   ou si q=1, R₁₇, R₁₈ et R₁₉ représentent chacun un atome d'hydrogène, ainsi que les sels formés par ces composés avec des bases ou des acides.
(6) Les polymères répondant à la formule générale (V) sont, par exemple, décrits dans le brevet français 1 400 366 : 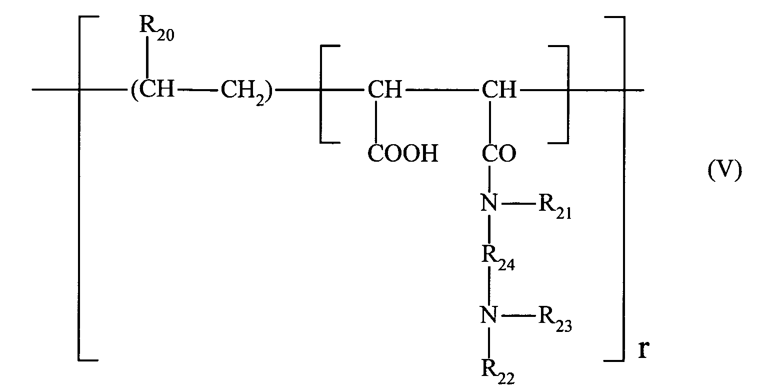 dans laquelle R₂₀ représente un atome d'hydrogène, un groupe CH₃O, CH₃CH₂O, phényle, R₂₁ désigne un atome d'hydrogène ou un groupe alkyle inférieur tel que méthyle, éthyle, R₂₂ désigne un atome d'hydrogène ou un groupe alkyle inférieur en C₁-C₆ tel que méthyle, éthyle, R₂₃ désigne un groupe alkyle inférieur en C₁-C₆ tel que méthyle, éthyle ou un groupe répondant à la formule : -R₂₄-N(R₂₂)₂, R₂₄ représentant un groupement -CH₂-CH₂-, -CH₂-CH₂-CH₂-, -CH₂-CH(CH₃)-, R₂₂ ayant les significations mentionnées ci-dessus.
(7) Les polymères dérivés de la N-carboxyalkylation du chitosane comme le N-carboxyméthyl-chitosane ou le N-carboxybutylchitosane, vendus sous la dénomination « EVALSAN » par la société JAN DEKKER.
(8) Les polymères amphotères du type -D-X-D-X choisis parmi:
   a) les polymères obtenus par action de l'acide chloracétique ou le chloracétate de sodium sur les composés comportant au moins un motif de formule :

      -D-X-D-X-D- (VI)

      où D désigne un groupe 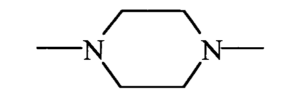 et X désigne le symbole E ou E', E ou E' identiques ou différents désignent un groupe bivalent qui est un groupe alkylène à chaîne droite ou ramifiée, comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques ; les atomes d'oxygène, d'azote et de soufre étant présents sous forme de groupements éther, thioéther, sulfoxyde, sulfone, sulfonium, alkylamine, alcénylamine, des groupements hydroxyle, benzylamine, oxyde d'amine, ammonium quaternaire, amide, imide, alcool, ester et/ou uréthanne.
   b) Les polymères de formule :

      -D-X-D-X- (VI')
   où D désigne un groupe 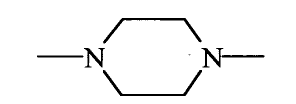 et X désigne le symbole E ou E' et au moins une fois E' ; E ayant la signification indiquée ci-dessus et E' est un groupe bivalent qui est un groupe alkylène à chaîne droite ou ramifiée, ayant jusqu'à 7 atomes de carbone dans la chaîne principale, substitué ou non par un ou plusieurs groupes hydroxyles et comportant un ou plusieurs atomes d'azote, l'atome d'azote étant substitué par une chaîne alkyle interrompue éventuellement par un atome d'oxygène et comportant obligatoirement une ou plusieurs fonctions carboxyles ou une ou plusieurs fonctions hydroxyles et bétaïnisées par réaction avec l'acide chloracétique ou du chloracétate de soude.
(9) les copolymères alkyl (C₁-C₅) vinyléther/anhydride maléique modifiés partiellement par semiamidification avec une N,N-dialkylaminoalkylamine telle que la N,N-diméthylaminopropylamine
ou par semiestérification avec un N,N-dialkylaminoalcanol. Ces copolymères peuvent également comporter d'autres comonomères vinyliques tels que le vinylcaprolactame.

Parmi les polymères fixants amphotères cités ci-dessus les plus particulièrement préférés selon l'invention, on citera ceux de la famille (3) tels que les copolymères dont la dénomination CTFA est Octylacrylamide/acrylates/butylamino-ethylmethacrylate copolymer, tels que les produits vendus sous les dénominations AMPHOMER®, AMPHOMER® LV 71 ou LOVOCRYL® 47 par la société NATIONAL STARCH et ceux de la famille (4) tels que les copolymères méthacrylate de méthyle/diméthyl-carboxyméthylammonio-éthylméthacrylate de méthyle vendu par exemple sous la dénomination DIAFORMER Z301 par la société SANDOZ.

Les polymères fixants non ioniques utilisables selon la présente invention sont choisis, par exemple, parmi :
- les polyalkyloxazolines ;
- les homopolymères d'acétate de vinyle ;
- les copolymères d'acétate de vinyle tels que, par exemple, les copolymères d'acétate de vinyle et d'ester acrylique, les copolymères d'acétate de vinyle et d'éthylène, ou les copolymères d'acétate de vinyle et d'ester maléïque, par exemple, de maléate de dibutyle ;
- les homopolymères et copolymères d'esters acryliques tels que, par exemple, les copolymères d'acrylates d'alkyle et de méthacrylates d'alkyle tels que les produits proposés par la société ROHM & HAAS sous les dénominations PRIMAL® AC-261 K et EUDRAGIT® NE 30 D, par la société BASF sous la dénomination 8845, par la société HOECHST sous la dénomination APPRETAN® N9212 ;
- les copolymères d'acrylonitrile et d'un monomère non ionique choisis, par exemple, parmi le butadiène et les (méth)acrylates d'alkyle ; on peut citer les produits proposés sous la dénomination CJ 0601 B par la société ROHM & HAAS ;
- les homopolymères de styrène ;
- les copolymères de styrène comme, par exemple, les copolymères de styrène et de (méth)acrylate d'alkyle tels que les produits MOWILITH® LDM 6911, MOWILITH® DM 611 et MOWILITH® LDM 6070 proposés par la société HOECHST, les produits RHODOPAS® SD 215 et RHODOPAS® DS 910 proposés par la société RHONE POULENC ; les copolymères de styrène, de méthacrylate d'alkyle et d'acrylate d'alkyle ; les copolymères de styrène et de butadiène ; ou les copolymères de styrène, de butadiène et de vinylpyridine ;
- les polyamides ;
- les homopolymères de vinyllactame différents des homopolymères de vinylpyrrolidone, tels que le polyvinylcaprolactame commercialisé sous la dénomination Luviskol® PLUS par la société BASF ; et
- les copolymères de vinyllactame tels qu'un copolymère poly(vinylpyrrolidone/vinyllactame) vendu sous le nom commercial Luvitec® VPC 55K65W par la société BASF, les copolymères poly(vinylpyrrolidone/acétate de vinyle) comme ceux commercialisés sous la dénomination PVPVA® S630L par la société ISP, Luviskol® VA 73, VA 64, VA 55, VA 37 et VA 28 par la société BASF ; et les terpolymères poly(vinylpyrrolidone/acétate de vinyle/propionate de vinyle) comme, par exemple, celui commercialisé sous la dénomination Luviskol® VAP 343 par la société BASF.

Les groupes alkyles des polymères non ioniques mentionnés ci-dessus ont, de préférence, de 1 à 6 atomes de carbone.

Selon l'invention, on peut également utiliser des polymères fixants de type siliconés greffés, comprenant une partie polysiloxane et une partie constituée d'une chaîne organique non siliconée, l'une des deux parties constituant la chaîne principale du polymère et l'autre étant greffée sur ladite chaîne principale.

Ces polymères sont par exemple décrits dans les demandes de brevet EP-A-0 412 704, EP-A-0 412 707, EP-A-0 640 105 et WO 95/00578, EP-A-0 582 152 et WO 93/23009 et les brevets US 4,693,935, US 4,728,571 et US 4,972,037.

Ces polymères peuvent être amphotères, anioniques ou non ioniques, et ils sont de préférence anioniques ou non ioniques.

De tels polymères sont, par exemple, les copolymères susceptibles d'être obtenus par polymérisation radicalaire à partir du mélange de monomères formé :
a) de 50 à 90 % en poids d'acrylate de tertiobutyle,
b) de 0 à 40 % en poids d'acide acrylique,
c) de 5 à 40 % en poids d'un macromère siliconé de formule 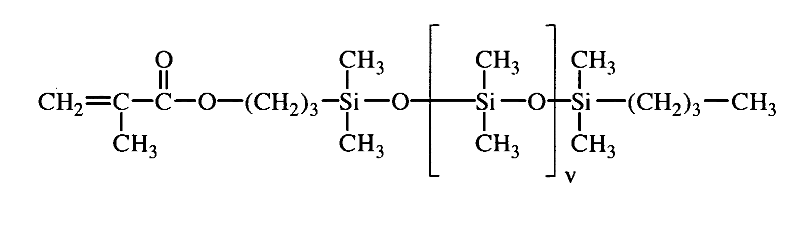
où v est un nombre allant de 5 à 700, les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type poly(acide (méth)acrylique) et du type poly((méth)acrylate d'alkyle), et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Comme autre type de polymères fixants siliconés, on peut citer le produit Luviflex® Silk commercialisé par la société BASF.

On peut également utiliser comme polymères fixants, des polyuréthanes fonctionnalisés ou non, siliconés ou non, cationiques, non-ioniques, anioniques ou amphotères, ou leurs mélanges.

Les polyuréthanes particulièrement visés par la présente invention sont ceux décrits dans les demandes EP 0 751 162, EP 0 637 600, EP 0 648 485 et FR 2 743 297 dont la demanderesse est titulaire, ainsi que dans les demandes EP 0 656 021 et WO 94/03510 de la société BASF, et EP 0 619 111 de la société National Starch.

Comme polyuréthanes convenant particulièrement bien dans la présente invention, on peut citer les produits commercialisés sous les dénominations LUVISET PUR® et LUVISET® Si PUR par la société BASF.

La concentration en polymère(s) fixant(s) additionnel(s) utilisé(s) dans les compositions selon la présente invention est comprise entre 0,1 et 20%, de préférence entre 0,5 et 10% en poids par rapport au poids total de la composition.

La composition cosmétique coiffante selon l'invention peut contenir en outre au moins un additif choisi parmi, les silicones sous forme soluble ou insolubles, dispersée, micro-dispersée, les polymères épaississants, les gélifiants, les agents tensio-actifs non-ioniques, anioniques, cationiques et amphotères, les céramides et pseudo-céramides, les vitamines et pro-vitamines dont le panthénol, les huiles végétales, animales, minérales et synthétiques, les cires autres que les céramides et pseudo-céramides, les filtres solaires hydrosolubles et liposolubles, siliconés ou non siliconés, le glycérol, les colorants permanents ou temporaires, les agents nacrants et opacifiants, les agents séquestrants, les agents plastifiants, les agents solubilisants, les agents modificateurs de pH, les agents épaississants minéraux, les agents anti-oxydants, les hydroxyacides, les agents de pénétration, les parfums, les agents solubilisants du parfum (peptisant), les agents conservateurs, les agents anti-corrosion.

Ces additifs sont présents dans la composition cosmétique selon l'invention en une quantité allant de 0 à 20 % en poids par rapport au poids total de la composition cosmétique.

L'homme de métier veillera à choisir les éventuels additifs et leur quantité de manière à ce qu'ils ne nuisent pas aux propriétés des compositions de la présente invention.

Avantageusement, les compositions selon la présente invention contiennent au moins un additif cosmétique additionnel choisi parmi les polymères épaississants, les gélifiants et les tensioactifs.

Selon un premier mode préféré, les compositions selon la présente invention contiennent au moins un polymère épaississant encore appelé " agents d'ajustement de la rhéologie".

Les agents d'ajustement de la rhéologie peuvent être choisis parmi les amides d'acides gras (diéthanol- ou monoéthanol-amide de coprah, monoéthanolamide d'acide alkyl éther carboxylique oxyéthyléné), les épaississants cellulosiques (hydroxyéthycellulose, hydroxypropylcellulose, carboxyméthylcellulose), la gomme de guar et ses dérivés (hydroxypropylguar), les gommes d'origine microbienne (gomme de xanthane, gomme de scléroglucane), les homopolymères ou copolymères réticulés d'acide acrylique ou d'acide acrylamidopropanesulfonique et les polymères épaississants associatifs tels que décrits ci-dessous.

Ces polymères associatifs sont des polymères hydrosolubles capables, dans un milieu aqueux, de s'associer réversiblement entre eux ou avec d'autres molécules.

Leur structure chimique comprend des zones hydrophiles, et des zones hydrophobes caractérisées par au moins une chaîne grasse.

Les polymères associatifs peuvent être de type anionique, cationique, amphotère ou non ionique.

Leur concentration peut varier d'environ 0,01 à 10%, de préférence 0,1 à 5% en poids par rapport au poids total de la composition selon l'invention.

Parmi les polymères associatifs de type anionique, on peut citer :
- (I) ceux comportant au moins un motif hydrophile, et au moins un motif éther d'allyle à chaîne grasse, plus particulièrement ceux dont le motif hydrophile est constitué par un monomère anionique insaturé éthylénique, plus particulièrement encore par un acide carboxylique vinylique et tout particulièrement par un acide acrylique ou un acide méthacrylique ou les mélanges de ceux ci, et dont le motif éther d'allyle à chaîne grasse correspond au monomère de formule (XV) suivante :

   CH₂ = C R' CH₂ O Bₙ R (XV)

   dans laquelle R' désigne H ou CH₃, B désigne le radical éthylèneoxy, n est nul ou désigne un entier allant de 1 à 100, R désigne un radical hydrocarboné choisi parmi les radicaux alkyl, arylalkyle, aryle, alkylaryle, cycloalkyle, comprenant de 8 à 30 atomes de carbone, de préférence 10 à 24, et plus particulièrement encore de 12 à 18 atomes de carbone. Un motif de formule (XV) plus particulièrement préféré est un motif dans lequel R' désigne H, n est égal à 10, et R désigne un radical stéaryl (C₁₈).
   Des polymères associatifs anioniques de ce type sont décrits et préparés, selon un procédé de polymérisation en émulsion, dans le brevet EP-0 216 479.
   Parmi ces polymères associatifs anioniques, on préfère particulièrement selon l'invention, les polymères formés à partir de 20 à 60% en poids d'acide acrylique et/ou d'acide méthacrylique, de 5 à 60% en poids de (méth)acrylates d'alkyles inférieurs, de 2 à 50% en poids d'éther d'allyl à chaîne grasse de formule (XV), et de 0 à 1% en poids d'un agent réticulant qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.
   Parmi ces derniers, on préfère tout particulièrement les terpolymères réticulés d'acide méthacrylique, d'acrylate d'éthyle, de polyéthylèneglycol (10 OE) éther d'alcool stéarylique (Steareth 10), notamment ceux vendus par la société ALLIED COLLOIDS sous les dénominations SALCARE SC80® et SALCARE SC90® qui sont des émulsions aqueuses à 30% d'un terpolymère réticulé d'acide méthacrylique, d'acrylate d'éthyle et de steareth-10-allyl éther (40/50/10).
- (II) ceux comportant au moins un motif hydrophile de type acide carboxylique insaturé oléfinique, et au moins un motif hydrophobe de type ester d'alkyl (C₁₀-C₃₀) d'acide carboxylique insaturé.
   De préférence, ces polymères sont choisis parmi ceux dont le motif hydrophile de type acide carboxylique insaturé oléfinique correspond au monomère de formule (XVI) suivante : 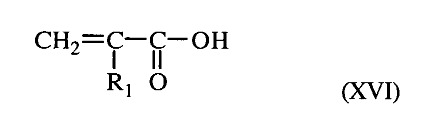 dans laquelle, R₁ désigne H ou CH₃ ou C₂H₅, c'est-à-dire des motifs acide acrylique, acide méthacrylique ou acide éthacrylique, et dont le motif hydrophobe de type ester d'alkyl (C₁₀-C₃₀) d'acide carboxylique insaturé correspond au monomère de formule (XVII) suivante 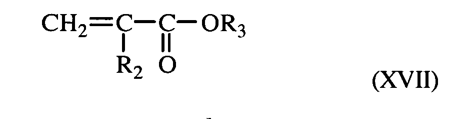 dans laquelle, R₂ désigne H ou CH₃ ou C₂H₅ (c'est-à-dire des motifs acrylates, méthacrylates ou éthacrylates) et de préférence H (motifs acrylates) ou CH₃ (motifs méthacrylates), R₃ désignant un radical alkyle en C₁₀-C₃₀, et de préférence en C₁₂-C₂₂.
   Des esters d'alkyles (C₁₀-C₃₀) d'acides carboxyliques insaturés conformes à l'invention comprennent par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.
   Des polymères anioniques de ce type sont par exemple décrits et préparés, selon les brevets US-3 915 921 et 4 509 949.
   Parmi ce type de polymères associatifs anioniques, on utilisera plus particulièrement des polymères formés à partir d'un mélange de monomères comprenant :
   (i) essentiellement de l'acide acrylique,
   (ii) un ester de formule (XVII) décrite ci-dessus et dans laquelle R₂ désigne H ou CH₃, R₃ désignant un radical alkyle ayant de 12 à 22 atomes de carbone, et
   (iii) un agent réticulant, qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.
      Parmi ce type de polymères associatifs anioniques, on utilisera plus particulièrement ceux constitués de 95 à 60% en poids d'acide acrylique (motif hydrophile), 4 à 40% en poids d'acrylate d'alkyles en C₁₀-C₃₀ (motif hydrophobe), et 0 à 6% en poids de monomère polymérisable réticulant, ou bien ceux constitués de 98 à 96% en poids d'acide acrylique (motif hydrophile), 1 à 4% en poids d'acrylate d'alkyles en C₁₀-C₃₀ (motif hydrophobe), et 0,1 à 0,6% en poids de monomère polymérisable réticulant tel que ceux décrits précédemment.
      Parmi lesdits polymères ci-dessus, on préfère tout particulièrement selon la présente invention, les produits vendus par la société GOODRICH sous les dénominations commerciales PEMULEN TR1®, PEMULEN TR2®, CARBOPOL 1382®, et encore plus préférentiellement le PEMULEN TR1®, et le produit vendu par la société S.E.P.P.I.C. sous la dénomination COATEX SX®.
- (III) les terpolymères d'anhydride maléique/α-oléfine en C₃₀-C₃₈/ maléate d'alkyle tel que le produit (copolymère anhydride maléique/α-oléfine en C₃₀-C₃₈/maléate d'isopropyle) vendu sous le nom PERFORMA V 1608® par la société NEWPHASE TECHNOLOGIES.
- (IV) les terpolymères acryliques comprenant :
   (a) environ 20% à 70% en poids d'un acide carboxylique à insaturation α,β-monoéthylénique,
   (b) environ 20 à 80% en poids d'un monomère à insaturation α,β-monoéthylénique non-tensio-actif différent de (a),
   (c) environ 0,5 à 60% en poids d'un mono-uréthane non-ionique qui est le produit de réaction d'un tensio-actif monohydrique avec un monoisocyanate à insaturation monoéthylénique,
   tels que ceux décrits dans la demande de brevet EP-A-0173109 et plus particulièrement celui décrit dans l'exemple 3, à savoir, un terpolymère acide méthacrylique /acrylate de méthyle/diméthyl métaisopropényl benzyl isocyanate d'alcool béhényle éthoxylé (400E) en dispersion aqueuse à 25%.
- (V) les copolymères comportant parmi leurs monomères un acide carboxylique à insaturation α,β-monoéthylénique et un ester d'acide carboxylique à insaturation α,β-monoéthylénique et d'un alcool gras oxyalkyléné.

Préférentiellement ces composés comprennent également comme monomère un ester d'acide carboxylique à insaturation α,β-monoéthylénique et d'alcool en C₁-C₄.

A titre d'exemple de ce type de composé on peut citer l'ACULYN 22® vendu par la société ROHM et HAAS, qui est un terpolymère acide méthacrylique/acrylate d'éthyle/méthacrylate de stéaryle oxyalkyléné.

Parmi les polymères associatifs de type cationique, on peut citer :
- (I) les polyuréthanes associatifs cationiques dont la famille a été décrite par la demanderesse dans la demande de brevet français N° 0009609; elle peut être représentée par la formule générale (XVIII) suivante :

   R-X-(P)n-[L-(Y)m]r-L'-(P')p-X'-R' (XVIII)

   dans laquelle :
   R et R', identiques ou différents, représentent un groupement hydrophobe ou un atome d'hydrogène ;
   X et X', identiques ou différents, représentent un groupement comportant une fonction amine portant ou non un groupement hydrophobe, ou encore le groupement L" ;
   L, L' et L", identiques ou différents, représentent un groupement dérivé d'un diisocyanate ;
   P et P', identiques ou différents, représentent un groupement comportant une fonction amine portant ou non un groupement hydrophobe ;
   Y représente un groupement hydrophile ;
   r est un nombre entier compris entre 1 et 100, de préférence entre 1 et 50 et en particulier entre 1 et 25,
   n, m, et p valent chacun indépendamment des autres entre 0 et 1000 ;
   la molécule contenant au moins une fonction amine protonée ou quaternisée et au moins un groupement hydrophobe.

   Dans un mode de réalisation préféré de ces polyuréthanes, les seuls groupements hydrophobes sont les groupes R et R' aux extrémités de chaîne.
   Une famille préférée de polyuréthanes associatifs cationiques est celle correspondant à la formule (XVIII) décrite ci-dessus et dans laquelle :
   R et R' représentent tous les deux indépendamment un groupement hydrophobe,
   X, X' représentent chacun un groupe L",
   n et p valent entre 1 et 1000 et
   L, L', L", P, P', Y et m ont la signification indiquée ci-dessus.

   Une autre famille préférée de polyuréthanes associatifs cationiques est celle correspondant à la formule (XVIII) ci-dessus dans laquelle :
   R et R' représentent tous les deux indépendamment un groupement hydrophobe, X, X' représentent chacun un groupe L", n et p valent 0, et L, L', L", Y et m ont la signification indiquée ci-dessus.

   Le fait que n et p valent 0 signifie que ces polymères ne comportent pas de motifs dérivés d'un monomère à fonction amine, incorporé dans le polymère lors de la polycondensation. Les fonctions amine protonées de ces polyuréthanes résultent de l'hydrolyse de fonctions isocyanate, en excès, en bout de chaîne, suivie de l'alkylation des fonctions amine primaire formées par des agents d'alkylation à groupe hydrophobe, c'est-à-dire des composés de type RQ ou R'Q, dans lequel R et R' sont tels que définis plus haut et Q désigne un groupe partant tel qu'un halogénure, un sulfate, etc.
   Encore une autre famille préférée de polyuréthanes associatifs cationiques est celle correspondant à la formule (Ia) ci-dessus dans laquelle :
   R et R' représentent tous les deux indépendamment un groupement hydrophobe,
   X et X' représentent tous les deux indépendamment un groupement comportant une amine quaternaire,
   n et p valent zéro, et
   L, L', Y et m ont la signification indiquée ci-dessus.

   La masse moléculaire moyenne en nombre des polyuréthanes associatifs cationiques est comprise de préférence entre 400 et 500 000, en particulier entre 1000 et 400 000 et idéalement entre 1000 et 300 000.
   Par groupement hydrophobe, on entend un radical ou polymère à chaîne hydrocarbonée, saturée ou non, linéaire ou ramifiée, pouvant contenir un ou plusieurs hétéroatomes tels que P, O, N, S, ou un radical à chaîne perfluorée ou siliconée. Lorsqu'il désigne un radical hydrocarboné, le groupement hydrophobe comporte au moins 10 atomes de carbone, de préférence de 10 à 30 atomes de carbone, en particulier de 12 à 30 atomes de carbone et plus préférentiellement de 18 à 30 atomes de carbone.
   Préférentiellement, le groupement hydrocarboné provient d'un composé monofonctionnel.
   A titre d'exemple, le groupement hydrophobe peut être issu d'un alcool gras tel que l'alcool stéarylique, l'alcool dodécylique, l'alcool décylique. Il peut également désigner un polymère hydrocarboné tel que par exemple le polybutadiène.
   Lorsque X et/ou X' désignent un groupement comportant une amine tertiaire ou quaternaire, X et/ou X' peuvent représenter l'une des formules suivantes : 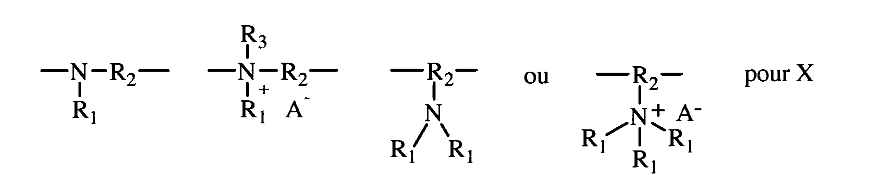 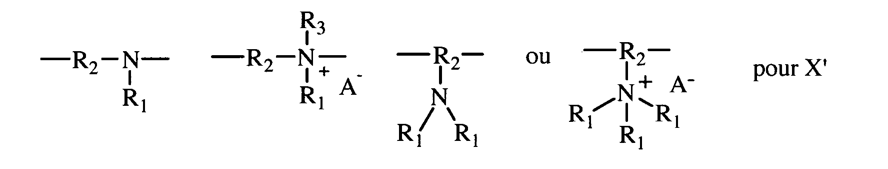 dans lesquelles :
   R₂ représente un radical alkylène ayant de 1 à 20 atomes de carbone, linéaire ou ramifié, comportant ou non un cycle saturé ou insaturé, ou un radical arylène, un ou plusieurs des atomes de carbone pouvant être remplacé par un hétéroatome choisi parmi N, S, O, P ;
   R₁ et R₃, identiques ou différents, désignent un radical alkyle
   ou alcényle en C₁-C₃₀, linéaire ou ramifié, un radical aryle, l'un au moins des atomes de carbone pouvant être remplacé par un hétéroatome choisi parmi N, S, O, P ;
   A⁻ est un contre-ion physiologiquement acceptable.

   Les groupements L, L' et L" représentent un groupe de formule : 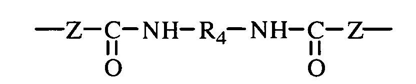 dans laquelle :
   Z représente -O-, -S- ou -NH- ; et
   R₄ représente un radical alkylène ayant de 1 à 20 atomes de carbone, linéaire ou ramifié, comportant ou non un cycle saturé ou insaturé, un radical arylène, un ou plusieurs des atomes de carbone pouvant être remplacé par un hétéroatome choisi parmi N, S, O et P.

   Les groupements P et P', comprenant une fonction amine peuvent représenter au moins l'une des formules suivantes : 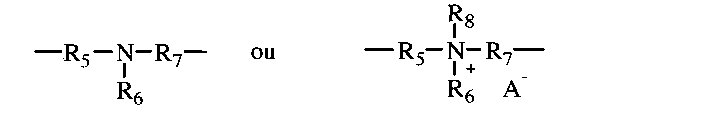 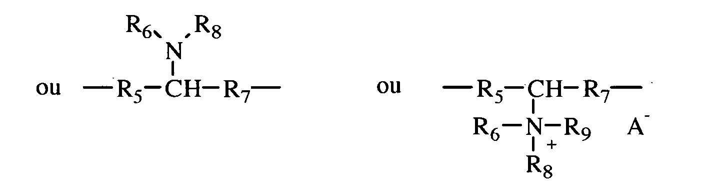 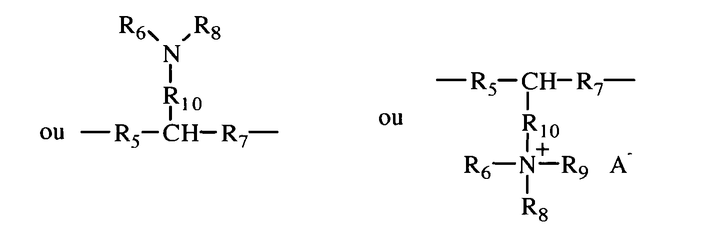 dans lesquelles :
   R₅ et R₇ ont les mêmes significations que R₂ défini précédemment;
   R₆, R₈ et R₉ ont les mêmes significations que R₁ et R₃ définis précédemment ;
   R₁₀ représente un groupe alkylène, linéaire ou ramifié, éventuellement insaturé et pouvant contenir un ou plusieurs hétéroatomes choisis parmi N, O, S et P,
   et A⁻ est un contre-ion physiologiquement acceptable.

   En ce qui concerne la signification de Y, on entend par groupement hydrophile, un groupement hydrosoluble polymérique ou non.
   A titre d'exemple, on peut citer, lorsqu'il ne s'agit pas de polymères, l'éthylèneglycol, le diéthylèneglycol et le propylèneglycol.
   Lorsqu'il s'agit, conformément à un mode de réalisation préféré, d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).
   Les polyuréthanes associatifs cationiques de formule (XVIII) utilisables selon l'invention sont formés à partir de diisocyanates et de différents composés possédant des fonctions à hydrogène labile. Les fonctions à hydrogène labile peuvent être des fonctions alcool, amine primaire ou secondaire ou thiol donnant, après réaction avec les fonctions diisocyanate, respectivement des polyuréthanes, des polyurées et des polythiourées. Le terme "polyuréthanes" utilisable selon la présente invention englobe ces trois types de polymères à savoir les polyuréthanes proprement dits, les polyurées et les polythiourées ainsi que des copolymères de ceux-ci.
   Un premier type de composés entrant dans la préparation du polyuréthane de formule (XVIII) est un composé comportant au moins un motif à fonction amine. Ce composé peut être multifonctionnel, mais préférentiellement le composé est difonctionnel, c'est-à-dire que selon un mode de réalisation préférentiel, ce composé comporte deux atomes d'hydrogène labile portés par exemple par une fonction hydroxyle, amine primaire, amine secondaire ou thiol. On peut également utiliser un mélange de composés multifonctionnels et difonctionnels dans lequel le pourcentage de composés multifonctionnels est faible.
   Comme indiqué précédemment, ce composé peut comporter plus d'un motif à fonction amine. Il s'agit alors d'un polymère portant une répétition du motif à fonction amine.
   Ce type de composés peut être représenté par l'une des formules suivantes :

   HZ-(P)n-ZH,

   ou

   HZ-(P')p-ZH

   dans lesquelles Z, P, P', n et p sont tels que définis plus haut.
   A titre d'exemple de composé à fonction amine, on peut citer la N-méthyldiéthanolamine, la N-tert-butyl-diéthanolamine, la N-sulfoéthyldiéthanolamine.
   Le deuxième composé entrant dans la préparation du polyuréthane de formule (XVIII) est un diisocyanate correspondant à la formule :

   O=C=N-R₄-N=C=O

   dans laquelle R₄ est défini plus haut.
   A titre d'exemple, on peut citer le méthylènediphényldiisocyanate, le méthylènecyclohexanediisocyanate, l'isophoronediisocyanate, le toluènediisocyanate, le naphtalènediisocyanate, le butanediisocyanate, l'hexanediisocyanate.
   Un troisième composé entrant dans la préparation du polyuréthane de formule (XVIII) est un composé hydrophobe destiné à former les groupes hydrophobes terminaux du polymère de formule (XVIII).
   Ce composé est constitué d'un groupe hydrophobe et d'une fonction à hydrogène labile, par exemple une fonction hydroxyle, amine primaire ou secondaire, ou thiol.
   A titre d'exemple, ce composé peut être un alcool gras, tel que notamment l'alcool stéarylique, l'alcool dodécylique, l'alcool décylique. Lorsque ce composé comporte une chaîne polymérique, il peut s'agir par exemple du polybutadiène hydrogéné alpha-hydroxyle.
   Le groupe hydrophobe du polyuréthane de formule (XVIII) peut également résulter de la réaction de quaternisation de l'amine tertiaire du composé comportant au moins un motif amine tertiaire. Ainsi, le groupement hydrophobe est introduit par l'agent quaternisant. Cet agent quaternisant est un composé de type RQ ou R'Q, dans lequel R et R' sont tels que définis plus haut et Q désigne un groupe partant tel qu'un halogénure, un sulfate etc.
   Le polyuréthane associatif cationique peut en outre comprendre une séquence hydrophile. Cette séquence est apportée par un quatrième type de composé entrant dans la préparation du polymère. Ce composé peut être multifonctionnel. Il est de préférence difonctionnel. On peut également avoir un mélange où le pourcentage en composé multifonctionnel est faible.
   Les fonctions à hydrogène labile sont des fonctions alcool, amine primaire ou secondaire, ou thiol. Ce composé peut être un polymère terminé aux extrémités des chaînes par l'une de ces fonctions à hydrogène labile.
   A titre d'exemple, on peut citer, lorsqu'il ne s'agit pas de polymères, l'éthylèneglycol, le diéthylèneglycol et le propylèneglycol.
   Lorsqu'il s'agit d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).
   Le groupe hydrophile noté Y dans la formule (XVIII) est facultatif. En effet, les motifs à fonction amine quaternaire ou protonée peuvent suffire à apporter la solubilité ou l'hydrodispersibilité nécessaire pour ce type de polymère dans une solution aqueuse.
   Bien que la présence d'un groupe Y hydrophile soit facultative, on préfère cependant des polyuréthanes associatifs cationiques comportant un tel groupe.
- (II) les dérivés de cellulose quaternisée et les polyacrylates à groupements latéraux aminés non cycliques.

Les dérivés de cellulose quaternisée sont en particulier,
- les celluloses quaternisées modifiées par des groupements comportant au moins une chaîne grasse, tels que les groupes alkyle, arylalkyle, alkylaryle comportant au moins 8 atomes de carbone, ou des mélanges de ceux-ci,
- les hydroxyéthylcelluloses quaternisées modifiées par des groupements comportant au moins une chaîne grasse, tels que les groupes alkyle, arylalkyle, alkylaryle comportant au moins 8 atomes de carbone, ou des mélanges de ceux-ci.

Les radicaux alkyle portés par les celluloses ou hydroxyéthylcelluloses quaternisées ci-dessus comportent de préférence de 8 à 30 atomes de carbone. Les radicaux aryle désignent de préférence les groupements phényle, benzyle, naphtyle ou anthryle.

On peut indiquer comme exemples d'alkylhydroxyéthylcelluloses quaternisées à chaînes grasses en C₈-C₃₀, les produits QUATRISOFT LM 200®, QUATRISOFT LM-X 529-18-A®, QUATRISOFT LM-X 529-18B® (alkyle en C₁₂) et QUATRISOFT LM-X 529-8® (alkyle en C₁₈) commercialisés par la société AMERCHOL et les produits CRODACEL QM®, CRODACEL QL® (alkyle en C12) et CRODACEL QS® (alkyle en C₁₈) commercialisés par la société CRODA.

Les polymères associatifs amphotères sont choisis de préférence parmi ceux comportant au moins un motif cationique non cyclique. Plus particulièrement encore, on préfère ceux préparés à partir ou comprenant 1 à 20 moles % de monomère comportant une chaîne grasse, et de préférence 1,5 à 15 moles% et plus particulièrement encore 1,5 à 6 moles %, par rapport au nombre total de moles de monomères.

Les polymères associatifs amphotères préférés selon l'invention comprennent, ou sont préparés en copolymérisant :
1) au moins un monomère de formule (XIX) ou (XX) : 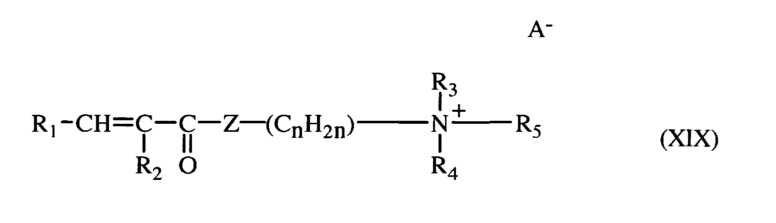 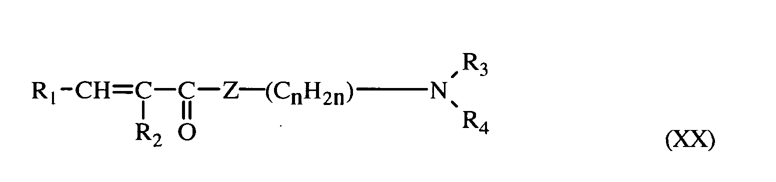 dans lesquelles, R₁ et R₂, identiques ou différents, représentent un atome d'hydrogène ou un radical méthyle, R₃, R₄ et R₅, identiques
   ou différents, représente un radical alkyle linéaire ou ramifié ayant de 1 à 30 atomes de carbone,
   Z représente un groupe NH ou un atome d'oxygène,
   n est un nombre entier de 2 à 5,
   A⁻ est un anion issu d'un acide organique ou minéral, tel qu'un anion méthosulfate ou un halogénure tel que chlorure ou bromure;
2) au moins un monomère de formule (XXI)

   R₆―CH=CR₇―COOH (XXI)

   dans laquelle, R₆ et R₇, identiques ou différents, représentent un atome d'hydrogène ou un radical méthyle;
   et
3) au moins un monomère de formule (XXII) :

   R₆―CH=CR₇―COXR₈ (XXII)
dans laquelle R₆ et R₇, identiques ou différents, représentent un atome d'hydrogène ou un radical méthyle, X désigne un atome d'oxygène ou d'azote et R₈ désigne un radical alkyle linéaire ou ramifié ayant de 1 à 30 atomes de carbone ;
l'un au moins des monomères de formule (XIX), (XX) ou (XXII) comportant au moins une chaîne grasse.

Les monomères de formule (XIX) et (XX) de la présente invention sont choisis, de préférence, dans le groupe constitué par :
- le diméthylaminoéthylméthacrylate, le diméthylaminoéthylacrylate,
- le diéthylaminoéthylméthacrylate, le diéthylaminoéthylacrylate,
- le diméthylaminopropylméthacrylate, le diméthylaminopropylacrylate,
- le diméthylaminopropylméthacrylamide, le diméthylaminopropylacrylamide,
ces monomères étant éventuellement quaternisés, par exemple par un halogénure d'alkyle en C₁-C₄ ou un sulfate de dialkyle en C₁-C₄.

Plus particulièrement, le monomère de formule (XIX) est choisi parmi le chlorure d'acrylamidopropyl triméthyl ammonium et le chlorure de méthacrylamidopropyl triméthyl ammonium.

Les monomères de formule (XXI) de la présente invention sont choisis, de préférence, dans le groupe constitué par l'acide acrylique, l'acide méthacrylique, l'acide crotonique et l'acide méthyl-2 crotonique. Plus particulièrement, le monomère de formule (XXI) est l'acide acrylique.

Les monomères de formule (XXII) de la présente invention sont choisis, de préférence, dans le groupe constitué par des acrylates ou méthacrylates d'alkyle en C₁₂-C₂₂ et plus particulièrement en C₁₆-C₁₈.

Les monomères constituant les polymères amphotères à chaîne grasse de l'invention sont de préférence déjà neutralisés et/ou quaternisés.

Le rapport du nombre de charges cationiques/charges anioniques est de préférence égal à environ 1.

Les polymères associatifs amphotères selon l'invention comprennent de préférence de 1 à 10 % moles du monomère comportant une chaîne grasse (monomère de formule (XIX), (XX) ou (XXII)), et de préférence de 1,5 à 6% moles.

Les poids moléculaires moyens en poids des polymères associatifs amphotères selon l'invention peuvent varier de 500 à 50.000.000 et sont de préférence compris entre 10.000 et 5 000 000.

Les polymères associatifs amphotères selon l'invention peuvent également contenir d'autres monomères tels que des monomères non ioniques et en particulier tels que les acrylates ou méthacrylates d'alkyle en C₁-C₄.

Des polymères associatifs amphotères selon l'invention sont par exemple décrits et préparés dans la demande de brevet WO9844012.

Parmi les polymères associatifs amphotères selon l'invention, on préfère les terpolymères acide acrylique/chlorure de (méth)acrylamidopropyl triméthyl ammonium/ méthacrylate de stéaryle.

Les polymères associatifs de type non ionique utilisables selon l'invention sont choisis de préférence parmi :
- (1) les celluloses modifiées par des groupements comportant au moins une chaîne grasse ;
   on peut citer à titre d'exemple :
   - les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en C₈-C₂₂, comme le produit NATROSOL PLUS GRADE 330 CS® (alkyles en C₁₆) vendu par la société AQUALON, ou le produit BERMOCOLL EHM 100® vendu par la société BEROL NOBEL,
   - celles modifiées par des groupes polyalkylène glycol éther d'alkyl phénol, tel que le produit AMERCELL POLYMER HM-1500® (polyéthylène glycol (15) éther de nonyl phénol) vendu par la société AMERCHOL.
- (2) les hydroxypropylguars modifiés par des groupements comportant au moins une chaîne grasse tel que le produit ESAFLOR HM 22® (chaîne alkyle en C₂₂) vendu par la société LAMBERTI, les produits RE210-18® (chaîne alkyle en C₁₄) et RE205-1® (chaîne alkyle en C₂₀) vendus par la société RHONE POULENC.
- (3) les copolymères de vinyl pyrrolidone et de monomères hydrophobes à chaîne grasse dont on peut citer à titre d'exemple :
   - les produits ANTARON V216® ou GANEX V216® (copolymère vinylpyrrolidone / hexadécène) vendu par la société I.S.P.
   - les produits ANTARON V220® ou GANEX V220® (copolymère vinylpyrrolidone / eicosène) vendu par la société I.S.P.
- (4) les copolymères de méthacrylates ou d'acrylates d'alkyles en C₁-C₆ et de monomères amphiphiles comportant au moins une chaîne grasse tels que par exemple le copolymère acrylate de méthyle/acrylate de stéaryle oxyéthyléné vendu par la société GOLDSCHMIDT sous la dénomination ANTIL 208®.
- (5) les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle.
- (6) les polyuréthanes polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.
- (7) les polymères à squelette aminoplaste éther possédant au moins une chaîne grasse, tels que les composés PURE THIX® proposés par la société SUD-CHEMIE.

De préférence, les polyéthers polyuréthanes comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.

Les polyéthers polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.

Les polyéthers polyuréthanes non-ioniques à chaîne grasse peuvent être des copolymères triblocs dont la séquence hydrophile est une chaîne polyoxyéthylénée comportant de 50 à 1000 groupements oxyéthylénés. Les polyéthers polyuréthanes non-ioniques comportent une liaison uréthanne entre les séquences hydrophiles, d'où l'origine du nom.

Par extension figurent aussi parmi les polyéthers polyuréthanes non-ioniques à chaîne grasse ceux dont les séquences hydrophiles sont liées aux séquences lipophiles par d'autres liaisons chimiques.

A titre d'exemples de polyéthers polyuréthanes non-ioniques à chaîne grasse utilisables dans l'invention, on peut aussi utiliser aussi le Rhéolate 205® à fonction urée vendu par la société RHEOX ou encore les Rhéolates® 208 , 204 ou 212, ainsi que l'Acrysol RM 184®.

On peut également citer le produit ELFACOS T210® à chaîne alkyle en C₁₂₋₁₄ et le produit ELFACOS T212® à chaîne alkyle en C₁₈ de chez AKZO.

Le produit DW 1206B® de chez ROHM & HAAS à chaîne alkyle en C₂₀ et à liaison uréthanne, proposé à 20 % en matière sèche dans l'eau, peut aussi être utilisé.

On peut aussi utiliser des solutions ou dispersions de ces polymères notamment dans l'eau ou en milieu hydroalcoolique. A titre d'exemple, de tels polymères on peut citer, le Rhéolate® 255, le Rhéolate® 278 et le Rhéolate® 244 vendus par la société RHEOX. On peut aussi utiliser le produit DW 1206F et le DW 1206J proposés par la société ROHM & HAAS.

Les polyéthers polyuréthanes utilisables selon l'invention sont en particulier ceux décrits dans l'article de G. Fonnum, J. Bakke et Fk. Hansen - Colloid Polym. Sci 271, 380.389 (1993).

Plus particulièrement encore on préfère utiliser un polyéther polyuréthane susceptible d'être obtenu par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate.

De tels polyéther polyuréthanes sont vendus notamment par la société ROHM & HAAS sous les appellations Aculyn 46® et Aculyn 44® [l'ACULYN 46® est un polycondensat de polyéthylèneglycol à 150 ou 180 moles d'oxyde d'éthylène, d'alcool stéarylique et de méthylène bis(4-cyclohexyl-isocyanate) (SMDI), à 15% en poids dans une matrice de maltodextrine (4%) et d'eau (81%); l'ACULYN 44® est un polycondensat de polyéthylèneglycol à 150 ou 180 moles d'oxyde d'éthylène, d'alcool décylique et de méthylène bis(4-cyclohexylisocyanate) (SMDI), à 35% en poids dans un mélange de propylèneglycol (39%) et d'eau (26%)].

A titre d'agents épaississants, les agents gélifiants non associatifs peuvent également être utilisés ; parmi ceux-ci on compte les polymères ou copolymères d'acides organiques carboxyliques insaturés ou d'esters insaturés, les dérivés polysaccharidiques, les gommes, les silicates colloïdaux, les polyéthylèneglycols, les polyvinylpyrrolidones, les gels de silice hydrophiles.

La quantité d'agents épaississants présents dans la composition selon l'invention est comprise entre 0,01 et 10% et de préférence entre 0,1 et 5% en poids par rapport au poids total de la composition.

Selon un deuxième mode préféré, la composition selon la présente invention contient en outre au moins un tensioactif.

Les tensioactifs convenant à la mise en oeuvre de la présente invention sont notamment les suivants :

### (i) Tensioactif(s) anionique(s) :

A titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkyl(C₆-C₂₄) sulfosuccinates, les alkyl(C₆-C₂₄) éthersulfosuccinates, les alkyl(C₆-C₂₄) amidesulfosuccinates ; les alkyl(C₆-C₂₄) sulfoacétates ; les acyl(C₆-C₂₄) sarcosinates et les acyl(C₆-C₂₄) glutamates. On peut également utiliser les esters d'alkyl(C₆-C₂₄)polyglycosides carboxyliques tels que les alkylglucoside citrates, les alkylpolyglycoside tartrate et les alkylpolyglycoside sulfosuccinates, les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser les acides d'alkyl D galactoside uroniques et leurs sels, les acides alkyl (C₆-C₂₄) éther carboxyliques polyoxyalkylénés, les acides alkyl(C₆-C₂₄) aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène en particulier d'éthylène, et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine.

### (iii) Tensioactif(s) amphotère(s) ou zwittérionique(s) :

Les agents tensioactifs amphotères ou zwitterioniques, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

R₂ -CONHCH₂CH₂ -N(R₃)(R₄)(CH₂COO-)

dans laquelle : R₂ désigne un radical alkyle d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₂'-CONHCH₂CH₂-N(B)(C)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO₃H
R₂' désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.

A titre d'exemple on peut citer le cocoamphodiacetate commercialisé sous la dénomination commerciale MIRANOL® C2M concentré par la société RHODIA CHIMIE.

### (iv) Tensioactifs cationiques :

Parmi les tensioactifs cationiques on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

La quantité d'agents tensioactifs présents dans la composition selon l'invention est comprise entre 0,01 et 40% et de préférence entre 0,1 et 30% en poids par rapport au poids total de la composition.

La présente demande concerne encore un procédé cosmétique pour la mise en forme ou le maintien de la coiffure comprenant la vaporisation sur les cheveux du contenu du dispositif aérosol selon la présente demande.

Lorsque le contenu du dispositif aérosol est vaporisé sur les cheveux, le dispositif aérosol selon l'invention peut générer un spray ou une mousse.

Les exemples suivants illustrent la présente invention et ne doivent être considérés en aucune manière comme limitant l'invention.

### Exemple 1 : Préparation d'un polymère pseudo-bloc poly (acide acrylique/acrylate de méthyle)

100 g d'acétate de butyle sont introduits dans un réacteur de 1 litre, puis on augmente la température de façon à passer de la température ambiante (25°C) à 90°C en 1 heure.

On ajoute ensuite, à 90°C et en 1 heure, 30 g d'acide acrylique, 30 g d'acrylate de méthyle, 40 g d'acétate de butyle, 70 g d'isopropanol et 1,8 g de 2.5- Bis(2-éthylhexanoylperoxy)-2.5-diméthylhexane (Trigonox® 141 d'Akzo Nobel).

Le mélange est maintenu 1 heure à 90°C.

On introduit ensuite au mélange précédent, toujours à 90°C et en 1 heure, 90 g d'acrylate de méthyle, 70 g d'acétate de butyle, 20 g d'isopropanol et 1,2 g de 2.5- Bis(2-éthylhexanoylperoxy)-2.5-diméthylhexane.

Le mélange est maintenu 3 heures à 90°C, puis dilué par 105 g d'acétate de butyle et 45 g d'isopropanol, puis l'ensemble est refroidi.

On obtient une solution à 40% de matière active en polymère dans le mélange acétate de butyle / isopropanol.

On obtient un polymère comprenant une première séquence ou bloc poly (acide acrylique/acrylate de méthyle) ayant une Tg de 80°C, une deuxième séquence polyacrylate de méthyle ayant une Tg de 10°C et une séquence intermédiaire qui est un polymère statistique acide acrylique/acrylate de méthyle/polyacrylate de méthyle.

Ce polymère présente une masse moyenne en poids de 50 000 g/Mol et une masse moyenne en nombre de 17 000, soit un indice de polydispersité I de 2,95.

Il a une température de transition vitreuse (Tg) de 49°C.

### Exemple 2 :

La demanderesse a préparé une composition coiffante propulsée par un gaz liquéfié dans un dispositif aérosol classique.

Données en pourcentages pondéraux par rapport au contenu total du dispositif aérosol, les proportions des différents ingrédients ont été reportées dans le tableau ci-dessous.

| | |
|---|---|
| Polymère pseudo bloc poly(acide acrylique/acrylate de méthyle)* | 3% M. A. |
| MEXOMERE PW (neutralisé à 100%) | 3% M. A. |
| Ethanol | 5% M.A. |
| Eau déminéralisée | 54 % |
| Diméthyl Ether | 35% |

| | |
|---|---|
| *préparé à l'exemple 1 | |

### Exemple 3 :

La demanderesse a préparé une mousse de coiffage:
Données en pourcentages pondéraux par rapport au contenu total du dispositif aérosol, les proportions des différents ingrédients ont été reportées dans le tableau ci-dessous.

| | |
|---|---|
| Polymère pseudo bloc poly(acide acrylique/ acrylate de méthyle)* | 2% M. A. |
| Monolaurate de sorbitane oxyéthyléné | 0,2% M.A. |
| Eau déminéralisée | 92,8% |
| Isobutane/propane (85/15) | 5% |

| | |
|---|---|
| *préparé à l'exemple 1 | |

### Exemple 4 :

La demanderesse a préparé une composition coiffante propulsée à l'air comprimé.

Données en pourcentages pondéraux par rapport au contenu total du dispositif aérosol, les proportions des différents ingrédients ont été reportées dans le tableau ci-dessous.

| | |
|---|---|
| Polymère pseudo bloc poly(acide acrylique/acrylate de méthyle)* | 3% M. A. |
| MEXOMERE PW (neutralisé à 100%) | 3% M. A. |
| Ethanol | 5% M.A. |
| Eau déminéralisée | 89 % |
| *préparé à l'exemple 1 | |

### Données en pourcentages pondéraux

Cette composition coiffante est conditionnée dans un dispositif aérosol à poche. Ce dispositif est commercialisé sous le nom de EP Spray, il comprend un ensemble constitué d'une poche soudée hermétiquement à une valve et d'un diffuseur à bus tourbillonnaire. La valve est fixée sur un bidon aérosol classique.

La poche est remplie avec la composition de l'exemple 4 et de l'air comprimé est introduit entre la poche et le bidon aérosol à une pression suffisante pour faire sortir le produit sous forme d'un spray.

La pression du gaz comprimé est comprise entre 1 et 12 bars, de préférence entre 9 et 11 bars.

## Revendications

1. Dispositif aérosol contenant un agent propulseur et une composition cosmétique coiffante comprenant, dans un milieu cosmétiquement acceptable contenant plus de 50% en poids d'eau par rapport au poids total de ladite composition, au moins un polymère pseudo-bloc et au moins un polymère fixant additionnel, ledit polymère pseudo-bloc comprenant au moins une première séquence et au moins une deuxième séquence incompatibles l'une avec l'autre et ayant des températures de transition vitreuse (Tg) différentes, lesdites première et deuxième séquences étant reliées entre elles par un segment intermédiaire comprenant au moins un monomère constitutif de la première séquence et au moins un monomère constitutif de la deuxième séquence et ledit polymère ayant un indice de polydispersité I supérieur à 2.

2. Dispositif selon la revendication 1, tel que le milieu cosmétiquement acceptable de la composition coiffante contient plus de 70% et de préférence plus de 85% en poids d'eau par rapport au poids total de la composition.

3. Dispositif selon l'une des revendications précédentes, **caractérisé en ce que** la première séquence du polymère pseudo-bloc est choisie parmi :
- a) une séquence ayant une Tg supérieure ou égale à 40°C,
- b) une séquence ayant une Tg inférieure ou égale à 20°C,
- c) une séquence ayant une Tg comprise entre 20 et 40°C, et
la deuxième séquence est choisie dans une catégorie a), b) ou c) différente de la première séquence.

4. Dispositif selon la revendication 3, **caractérisé en ce que** la séquence ayant une Tg supérieure ou égale à 40°C est issue en totalité ou en partie d'un ou plusieurs monomères, qui sont tels que l'homopolymère préparé à partir de ces monomères a une température de transition vitreuse supérieure ou égale à 40°C.

5. Dispositif selon la revendication 4, **caractérisé en ce que** le polymère pseudo-bloc est tel que les monomères dont l'homopolymère correspondant a une température de transition vitreuse supérieure ou égale à 40°C sont choisis parmi les monomères suivants :
- les méthacrylates de formule CH₂ = C(CH₃)-COOR₁
dans laquelle R₁ représente un groupe alkyle non substitué, linéaire ou ramifié, contenant de 1 à 4 atomes de carbone, tel qu'un groupe méthyle, éthyle, propyle ou isobutyle ou R₁ représente un groupe cycloalkyle C₄ à C₁₂,
- les acrylates de formule CH₂ = CH-COOR₂
dans laquelle R₂ représente un groupe cycloalkyle en C₄ à C₁₂ tel que l'acrylate d'isobornyle ou un groupe tertio butyle,
- les (méth)acrylamides de formule : 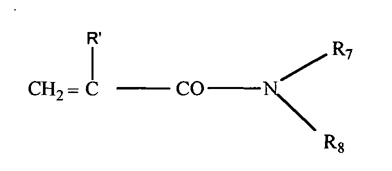 où R₇ et R₈ identiques ou différents représentent chacun un atome d'hydrogène ou un groupe alkyle de 1 à 12 atomes de carbone linéaire ou ramifié, tel qu'un groupe n-butyle, t-butyle, isopropyle, isohexyle, isooctyle, ou isononyle ; ou R₇ représente H et R₈ représente un groupement 1,1-diméthyl-3-oxobutyl, et R' désigne H ou méthyle.
- et leurs mélanges.

6. Dispositif selon la revendication 4 ou 5, **caractérisé en ce que** le polymère pseudo-bloc est tel que les monomères dont l'homopolymère correspondant a une température de transition vitreuse supérieure ou égale à 40°C sont choisis parmi le méthacrylate de méthyle, le méthacrylate d'isobutyle, le (méth)acrylate d'isobornyle et leurs mélanges.

7. Dispositif selon la revendication 3, **caractérisé en ce que** la séquence ayant une Tg inférieure ou égale à 20°C est issue en totalité
ou en partie d'un ou plusieurs monomères, qui sont tel(s) que l'homopolymère préparé à partir de ces monomères a une température de transition vitreuse inférieure ou égale à 20°C.

8. Dispositif selon la revendication 7, **caractérisé en ce que** les monomères dont l'homopolymère correspondant à une température de transition vitreuse inférieure ou égale à 20°C sont choisis parmi les monomères suivants :
- les acrylates de formule CH₂ = CHCOOR₃,
R₃ représentant un groupe alkyle non substitué en C₁ à C₁₂, linéaire ou ramifié, à l'exception du groupe tertiobutyle, dans lequel se trouve(nt) éventuellement intercalé(s) un ou plusieurs hétéroatomes choisis parmi O, N, S ;
- les méthacrylates de formule CH₂ = C(CH₃)-COOR₄;
R₄ représentant un groupe alkyle non substitué en C₆ à C₁₂ linéaire ou ramifié, dans lequel se trouve(nt) éventuellement intercalé(s) un ou plusieurs hétéroatomes choisis parmi 0, N et S;
- les esters de vinyle de formule R₅-CO-O-CH = CH₂
où R₅ représente un groupe alkyle en C₄ à C₁₂ linéaire ou ramifié ;
- les éthers d'alcool vinylique et d'alcool en C₄ à C₁₂ ;
- les N-alkyl en C₄ à C₁₂ acrylamides, tels que le N-octylacrylamide,
- et leurs mélanges.

9. Dispositif selon la revendication 7 ou 8, **caractérisé en ce que** le polymère pseudo-bloc est tel que les monomères dont l'homopolymère correspondant a une température de transition vitreuse inférieure ou égale à 20°C sont choisis parmi les acrylates d'alkyle dont la chaîne alkyle comprend de 1 à 10 atomes de carbone, à l'exception du groupe tertiobutyle.

10. Dispositif selon la revendication 3, **caractérisé en ce que** la séquence ayant une Tg comprise entre 20 et 40°C est issue en totalité ou en partie de un ou de plusieurs monomères, qui sont tel(s) que l'homopolymère préparé à partir de ces monomères a une température de transition vitreuse comprise entre 20 et 40°C.

11. Dispositif selon la revendication 10, **caractérisé en ce que** la séquence ayant une Tg comprise entre 20 et 40°C est issue en totalité ou en partie de monomères qui sont tel(s) que l'homopolymère correspondant a une Tg supérieure ou égale à 40°C et de monomères qui sont tel(s) que l'homopolymère correspondant a une Tg inférieure ou égale à 20°C.

12. Dispositif selon la revendication 10 ou 11, **caractérisé en ce que** la séquence ayant une Tg comprise entre 20 et 40°C est issue en totalité ou en partie de monomères choisis parmi le méthacrylate de méthyle, l'acrylate et le méthacrylate d'isobornyle, le méthacrylate de trifuoroéthyle, l'acrylate de butyle, l'acrylate d'éthyl-2 hexyle et leurs mélanges.

13. Dispositif selon la revendication 1 contenant un agent propulseur et une composition cosmétique coiffante comprenant, dans un milieu cosmétiquement acceptable contenant plus de 50% en poids d'eau par rapport au poids total de la composition cosmétique coiffante au moins un polymère pseudo-bloc et au moins un polymère additionnel, ledit polymère pseudo-bloc comprenant au moins une première séquence et au moins une deuxième séquence incompatibles l'une avec l'autre, la première séquence ayant une température de transition vitreuse (Tg) supérieure ou égale à 40°C et la deuxième séquence ayant une température de transition vitreuse inférieure ou égale à 20°C, lesdites première et deuxième séquences étant reliées entre elles par un segment intermédiaire comprenant au moins un monomère constitutif de la première séquence et au moins un monomère constitutif de la deuxième séquence et ledit polymère ayant un indice de polydispersité I supérieur à 2.

14. Dispositif aérosol selon la revendication 13, **caractérisé en ce que** la première séquence du polymère pseudo-bloc est issue en totalité ou en partie de un ou plusieurs monomères, qui sont tels que l'homopolymère préparé à partir de ces monomères a une température de transition vitreuse supérieure ou égale à 40°C.

15. Dispositif aérosol selon la revendication 13 ou 14, **caractérisé en ce que** la première séquence du polymère pseudo-bloc est un copolymère issu de monomères qui sont tels que l'homopolymère préparé à partir de ces monomères a une température de transition vitreuse supérieure ou égale à 40°C.

16. Dispositif aérosol selon l'une des revendications 13 à 15, **caractérisé en ce que** le polymère pseudo-bloc est tel que les monomères dont l'homopolymère correspondant a une température de transition vitreuse supérieure ou égale à 40°C sont choisis parmi les monomères suivants :
- les méthacrylates de formule CH₂ = C(CH₃)-COOR₁
dans laquelle R₁ représente un groupe alkyle non substitué, linéaire ou ramifié, contenant de 1 à 4 atomes de carbone, tel qu'un groupe méthyle, éthyle, propyle ou isobutyle ou R₁ représente un groupe cycloalkyle C₄ à C₁₂,
- les acrylates de formule CH₂ = CH-COOR₂
dans laquelle R₂ représente un groupe cycloalkyle en C₄ à C₁₂ tel que un groupe isobornyle ou un groupe tertio butyle,
- les (méth)acrylamides de formule : 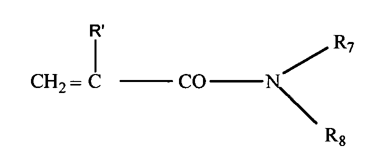 où R₇ et R₈ identiques ou différents représentent chacun un atome d'hydrogène ou un groupe alkyle de 1 à 12 atomes de carbone linéaire ou ramifié, tel qu'un groupe n-butyle, t-butyle, isopropyle, isohexyle, isooctyle, ou isononyle ; ou R₇ représente H et R₈ représente un groupement 1,1-diméthyl-3-oxobutyl, et R' désigne H ou méthyle,
- et leurs mélanges.

17. Dispositif aérosol selon l'une des revendications 13 à 16, **caractérisé en ce que** le polymère pseudo-bloc est tel que les monomères dont l'homopolymère correspondant à une température de transition vitreuse supérieure ou égale à 40°C sont choisis parmi le méthacrylate de méthyle, le méthacrylate d'isobutyle, le (méth)acrylate d'isobornyle et leurs mélanges.

18. Dispositif aérosol selon l'une des revendications 13 à 17, **caractérisé en ce que** la proportion de la première séquence va de 20 à 90% en poids du polymère, mieux de 30 à 80% et encore mieux de 50 à 70%.

19. Dispositif aérosol selon l'une des revendications 13 à 18, **caractérisé en ce que** le polymère pseudo-bloc est tel que la deuxième séquence est issue en totalité ou en partie de un ou plusieurs monomères, qui sont tel(s) que l'homopolymère préparé à partir de ces monomères a une température de transition vitreuse inférieure ou égale à 20°C.

20. Dispositif aérosol selon la revendication 19, **caractérisé en ce que** le polymère pseudo-bloc est tel que la deuxième séquence est un homopolymère issu de monomères qui sont tel(s) que l'homopolymère préparé à partir de ces monomères a une température de transition vitreuse inférieure ou égale à 20°C.

21. Dispositif aérosol selon la revendication 19 ou 20, **caractérisé en ce que** le polymère pseudo-bloc est tel que les monomères dont l'homopolymère correspondant à une température de transition vitreuse inférieure ou égale à 20°C sont choisis parmi les monomères suivants :
- les acrylates de formule CH₂ = CHCOOR₃,
R₃ représentant un groupe alkyle non substitué en C₁ à C₁₂, linéaire ou ramifié, à l'exception du groupe tertiobutyle, dans lequel se trouve(nt) éventuellement intercalé(s) un ou plusieurs hétéroatomes choisis parmi O, N, S ;
- les méthacrylates de formule CH₂ = C(CH₃)-COOR₄,
R₄ représentant un groupe alkyle non substitué en C₆ à C₁₂ linéaire ou ramifié, dans lequel se trouve(nt) éventuellement intercalé(s) un ou plusieurs hétéroatomes choisis parmi 0, N et S;
- les esters de vinyle de formule R₅-CO-O-CH = CH₂
où R₅ représente un groupe alkyle en C₄ à C₁₂ linéaire ou ramifié ;
- les éthers d'alcool vinylique et d'alcool en C₄ à C₁₂ ;
- les N-alkyl en C₄ à C₁₂ acrylamides, tels que le N-octylacrylamide,
- et leurs mélanges.

22. Dispositif aérosol selon l'une des revendications 19 à 21, **caractérisé en ce que** le polymère pseudo-bloc est tel que les monomères dont l'homopolymère correspondant à une température de transition vitreuse inférieure ou égale à 20°C sont choisis parmi les acrylates d'alkyle dont la chaîne alkyle comprend de 1 à 10 atomes de carbone, à l'exception du groupe tertiobutyle.

23. Dispositif aérosol selon l'une des revendications 19 à 22, **caractérisé en ce que** la proportion de la deuxième séquence ayant une Tg inférieure ou égale à 20°C va de 5 à 75% en poids du polymère, mieux de 15 à 50% et encore mieux de 25 à 45%.

24. Dispositif selon la revendication 1 contenant un agent propulseur et une composition cosmétique coiffante comprenant, dans un milieu cosmétiquement acceptable contenant plus de 50% en poids d'eau par rapport au poids total de la composition cosmétique coiffante, au moins un polymère pseudo bloc et au moins un polymère fixant additionnel, ledit polymère pseudo-bloc comprenant au moins une première séquence et au moins une deuxième séquence incompatibles l'une avec l'autre, la première séquence ayant une température de transition vitreuse (Tg) comprise entre 20 et 40°C et la deuxième séquence ayant une température de transition vitreuse inférieure ou égale à 20°C ou une température de transition vitreuse supérieure ou égale à 40°C, lesdites première et deuxième séquences étant reliées entre elles par un segment intermédiaire comprenant au moins un monomère constitutif de la première séquence et au moins un monomère constitutif de la deuxième séquence et ledit polymère ayant une indice de polydispersité I supérieur à 2.

25. Dispositif aérosol selon la revendication 24, **caractérisé en ce que** le polymère pseudo-bloc est tel que la première séquence ayant une Tg comprise entre 20 et 40°C est issue en totalité ou en partie d'un ou de plusieurs monomères, qui sont tel(s) que l'homopolymère préparé à partir de ces monomères a une température de transition vitreuse comprise entre 20 et 40°C.

26. Dispositif aérosol selon l'une des revendications 24 ou 25, **caractérisé en ce que** le polymère pseudo-bloc est tel que la première séquence ayant une Tg comprise entre 20 et 40°C est un copolymère issu de monomères qui sont tel(s) que l'homopolymère correspondant a une Tg supérieure ou égale à 40°C et de monomères qui sont tel(s) que l'homopolymère correspondant a une Tg inférieure ou égale à 20°C.

27. Dispositif aérosol selon l'une des revendications 24 à 26, **caractérisé en ce que** le polymère pseudo-bloc est tel que la première séquence ayant une Tg comprise entre 20 et 40°C est issue de monomères choisis parmi le méthacrylate de méthyle, l'acrylate et le méthacrylate d'isobornyle, l'acrylate de butyle, l'acrylate d'éthyl 2-hexyle et leurs mélanges.

28. Dispositif aérosol selon l'une des revendications 24 à 27, **caractérisé en ce que** le polymère pseudo-bloc est tel que la proportion de la première séquence ayant une Tg comprise entre 20 et 40°C va de 10 à 85% en poids du polymère, mieux de 30 à 80% et encore mieux de 50 à 70%.

29. Dispositif aérosol selon l'une des revendications 24 à 28, **caractérisé en ce que** le polymère pseudo-bloc est tel que la deuxième séquence a une Tg supérieure ou égale à 40°C et est issue en totalité ou en partie de un ou plusieurs monomères, qui sont tels que l'homopolymère préparé à partir de ces monomères a une température de transition vitreuse supérieure ou égale à 40°C.

30. Dispositif aérosol selon l'une des revendications 24 à 29, **caractérisé en ce que** le polymère pseudo-bloc est tel que la deuxième séquence a une Tg supérieure ou égale à 40°C et est un homopolymère issu de monomères, qui sont tels que l'homopolymère préparé à partir de ces monomères a une température de transition vitreuse supérieure ou égale à 40°C.

31. Dispositif aérosol selon l'une des revendications 29 ou 30, **caractérisé en ce que** le polymère pseudo-bloc est tel que les monomères dont l'homopolymère correspondant à une température de transition vitreuse supérieure ou égale à 40°C sont choisis parmi les monomères suivants :
- les méthacrylates de formule CH₂ = C(CH₃)-COOR₁
dans laquelle R₁ représente un groupe alkyle non substitué, linéaire ou ramifié, contenant de 1 à 4 atomes de carbone, tel qu'un groupe méthyle, éthyle, propyle ou isobutyle ou R₁ représente un groupe cycloalkyle C₄ à C₁₂,
- les acrylates de formule CH₂ = CH-COOR₂
dans laquelle R₂ représente un groupe cycloalkyle en C₄ à C₁₂ tel que un groupe isobornyle ou un groupe tertio butyle,
- les (méth)acrylamides de formule : 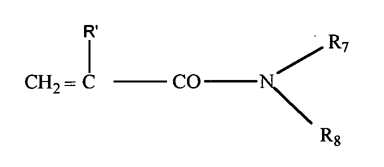 où R₇ et R₈ identiques ou différents représentent chacun un atome d'hydrogène ou un groupe alkyle de 1 à 12 atomes de carbone linéaire ou ramifié, tel qu'un groupe n-butyle, t-butyle, isopropyle, isohexyle, isooctyle, ou isononyle ; ou R₇ représente H et R₈ représente un groupement 1,1-diméthyl-3-oxobutyl, et R' désigne H ou méthyle,
- et leurs mélanges.

32. Dispositif aérosol selon l'une des revendications 29 à 31, **caractérisé en ce que** le polymère pseudo-bloc est tel que les monomères dont l'homopolymère correspondant à une température de transition vitreuse supérieure ou égale à 40°C sont choisis parmi le méthacrylate de méthyle, le méthacrylate d'isobutyle, le (méth)acrylate d'isobornyle et leurs mélanges.

33. Dispositif aérosol selon l'une des revendications 29 à 32, **caractérisé en ce que** le polymère pseudo-bloc est tel que la proportion de la deuxième séquence ayant une Tg supérieure ou égale à 40°C va de 10 à 85%, de préférence de 20 à 70% et mieux de 30 à 70% en poids du polymère.

34. Dispositif aérosol selon l'une des revendications 24 à 28, **caractérisé en ce que** le polymère pseudo-bloc est tel que la deuxième séquence a une Tg inférieure ou égale à 20°C et est issue en totalité ou en partie de un ou plusieurs monomères, qui sont tel(s) que l'homopolymère préparé à partir de ces monomères a une température de transition vitreuse inférieure ou égale à 20°C.

35. Dispositif aérosol selon l'une des revendications 24 à 28, **caractérisée en ce que** le polymère pseudo-bloc est tel que la deuxième séquence a une Tg inférieure ou égale à 20°C et est un homopolymère issu de monomères qui sont tel(s) que l'homopolymère préparé à partir de ces monomères a une température de transition vitreuse inférieure ou égale à 20°C

36. Dispositif aérosol selon la revendication 34 ou 35, **caractérisé en ce que** le polymère pseudo-bloc est tel que les monomères dont l'homopolymère correspondant à une température de transition vitreuse inférieure ou égale à 20°C sont choisis parmi les monomères suivants :
- les acrylates de formule CH₂ = CHCOOR₃,
R₃ représentant un groupe alkyle non substitué en C₁ à C₁₂, linéaire où ramifié, à l'exception du groupe tertiobutyle, dans lequel se trouve(nt) éventuellement intercalé(s) un ou plusieurs hétéroatomes choisis parmi O, N, S ;
- les méthacrylates de formule CH₂ = C(CH₃)-COOR₄,
R₄ représentant un groupe alkyle non substitué en C₆ à C₁₂ linéaire ou ramifié, dans lequel se trouve(nt) éventuellement intercalé(s) un ou plusieurs hétéroatomes choisis parmi 0, N et S;
- les esters de vinyle de formule R₅-CO-O-CH = CH₂
où R₅ représente un groupe alkyle en C₄ à C₁₂ linéaire ou ramifié ;
- les éthers d'alcool vinylique et d'alcool en C₄ à C₁₂, éthyl ;
- les N-alkyl en C₄ à C₁₂ acrylamides, tels que le N-octylacrylamide,
- et leurs mélanges.

37. Dispositif aérosol selon l'une des revendications 34 à 36, **caractérisé en ce que** le polymère pseudo-bloc est tel que les monomères dont les homopolymères ont des températures de transition vitreuse inférieures ou égales à 20°C sont choisis parmi les acrylates d'alkyle dont la chaîne alkyle comprend de 1 à 10 atomes de carbone, à l'exception du groupe tertiobutyle.

38. Dispositif aérosol selon l'une des revendications 34 à 37, **caractérisé en ce que** le polymère pseudo-bloc est tel que la proportion de la séquence ayant une température de transition vitreuse inférieure ou égale à 20°C va de 20 à 90% en poids du polymère, mieux de 30 à 80% et encore mieux de 50 à 70%.

39. Dispositif aérosol selon l'une des revendications précédentes, **caractérisé en ce que** le polymère pseudo-bloc est tel que la première séquence et/ou la deuxième séquence comprend au moins un monomère additionnel.

40. Dispositif aérosol selon la revendication 39, **caractérisé en ce que** le polymère pseudo-bloc est tel que le monomère additionnel est choisi parmi les monomères hydrophiles, les monomères à insaturation éthylénique comprenant un ou plusieurs atomes de silicium et leurs mélanges.

41. Dispositif aérosol selon la revendication 39 ou 40, **caractérisée en ce que** le polymère pseudo-bloc est tel que le monomère additionnel est choisi parmi :
- les monomères à insaturation(s) éthylénique(s) comprenant au moins une fonction acide carboxylique ou sulfonique
- les méthacrylates de formule CH₂ = C(CH₃)-COOR₆
dans laquelle R₆ représente un groupe alkyle linéaire ou ramifié, contenant de 1 à 4 atomes de carbone, tel qu'un groupe méthyle, éthyle, propyle ou isobutyle, ledit groupe alkyle étant substitué par un ou plusieurs substituants choisis parmi les groupes hydroxyle (comme le méthacrylate de 2-hydroxypropyle, le méthacrylate de 2-hydroxyéthyle) et les atomes d'halogènes (Cl, Br, I, F), tel que le méthacrylate de trifluoroéthyle,
- les méthacrylates de formule CH₂ = C(CH₃)-COOR₉,
R₉ représentant un groupe alkyle en C₆ à C₁₂ linéaire ou ramifié, dans lequel se trouve(nt) éventuellement intercalé(s) un ou plusieurs hétéroatomes choisis parmi 0, N et S, ledit groupe alkyle étant substitué par un ou plusieurs substituants choisis parmi les groupes hydroxyle et les atomes d'halogènes (Cl, Br, I, F) ;
- les acrylates de formule CH₂ = CHCOOR₁₀,
R₁₀ représentant un groupe alkyle en C₁ à C₁₂ linéaire ou ramifié substitué par un ou plusieurs substituants choisis parmi les groupes hydroxyle et les atomes d'halogène (Cl, Br, I et F), tel que l'acrylate de 2-hydroxypropyle et l'acrylate de 2-hydroxyéthyle, ou R₁₀ représente un alkyle en C₁ à C₁₂-O-POE (polyoxyéthylène) avec répétition du motif oxyéthylène de 5 à 30 fois, par exemple méthoxy-POE, ou R₈ représente un groupement polyoxyéthylèné comprenant de 5 à 30 motifs d'oxyde d'éthylène

42. Dispositif aérosol selon l'une des revendications 39 à 41, **caractérisé en ce que** le polymère pseudo-bloc est tel que le ou les monomères additionnels sont choisis parmi l'acide acrylique, l'acide méthacrylique, le méthacrylate de trifluoroéthyle et leurs mélanges.

43. Dispositif aérosol selon l'une des revendications 39 à 42, **caractérisé en ce que** le polymère pseudo-bloc est tel que le ou les monomères additionnel(s) représente(nt) de 1 à 30% en poids du poids total des première et/ou deuxième séquences.

44. Dispositif aérosol selon l'une des revendications précédentes, **caractérisé en ce que** le polymère pseudo-bloc est tel que chacune des première et deuxième séquence comprend au moins un monomère choisi parmi les esters d'acide (meth)acrylique, et éventuellement au moins un monomère choisi parmi l'acide (meth)acrylique et leurs mélanges.

45. Dispositif aérosol selon l'une des revendications précédentes, **caractérisé en ce que** chacune des première et deuxième séquence est issue en totalité d'au moins un monomère choisi parmi l'acide acrylique, les esters d'acide (méth)acrylique, et éventuellement d'au moins un monomère choisi parmi l'acide (méth)acrylique, et leurs mélanges.

46. Dispositif aérosol selon l'une des revendications précédentes, **caractérisé en ce** le polymère pseudo-bloc est tel que l'écart entre les températures de transition vitreuse (Tg) des première et deuxième séquences est supérieur à 10°C, mieux, supérieur à 20°C, de préférence supérieure à 30°C et mieux supérieure à 40°C.

47. Dispositif aérosol selon l'une des revendications précédentes, **caractérisé en ce que** le polymère pseudo-bloc est tel que la séquence intermédiaire a une température de transition vitreuse comprise entre les températures de transition vitreuse des première et deuxième séquences.

48. Dispositif aérosol selon l'une des revendications précédentes, **caractérisé en ce que** le polymère pseudo-bloc est tel qu'il a un indice de polydispersité supérieur ou égal à 2,5, de préférence supérieure ou égal à 2,8.

49. Dispositif aérosol selon l'une des revendications précédentes, **caractérisé en ce que** le polymère a un indice de polydispersité compris entre 2,8 et 6.

50. Dispositif aérosol selon l'une des revendications précédentes, **caractérisé en ce que** la masse moyenne en poids (Mw) du polymère est inférieure ou égale à 300 000.

51. Dispositif aérosol selon l'une des revendications précédentes, **caractérisé en ce que** la masse moyenne en poids (Mw) du polymère va de 35 000 à 200 000, et mieux de 45 000 à 150 000.

52. Dispositif aérosol selon l'une des revendications précédentes, **caractérisé en ce que** la masse moyenne en nombre (Mn) du polymère est inférieure ou égale à 70 000.

53. Dispositif aérosol selon l'une des revendications précédentes, **caractérisé en ce que** la masse moyenne en nombre (Mn) du polymère va de 10 000 à 60 000, et mieux de 12 000 à 50 000.

54. Dispositif aérosol selon l'une des revendications précédentes, **caractérisé en ce que** le polymère n'est pas soluble dans l'eau ou dans un mélange d'eau et de monoalcools inférieurs linéaires
ou ramifiés ayant de 2 à 5 atomes de carbone, sans modification de pH, à une teneur en matière active d'au moins 1% en poids, à température ambiante (25°C).

55. Dispositif aérosol selon l'une des revendications précédentes, **caractérisé en ce que** le polymère n'est pas un élastomère.

56. Dispositif aérosol selon l'une des revendications précédentes, **caractérisé en ce que** le polymère est un polymère éthylénique séquencé linéaire filmogène.

57. Dispositif aérosol selon l'une des revendications précédentes, **caractérisé en ce qu'**elle comprend de 0,1 à 60 % en poids en matière active de polymère pseudo-bloc, de préférence de 5 % à 50% en poids, et de préférence encore de 10 à 40 % en poids.

58. Dispositif aérosol selon l'une des revendications précédentes tel que le polymère fixant additionnel est choisi parmi les polymères fixants anioniques, cationiques, amphotères, non-ioniques, ou leurs mélanges.

59. Dispositif aérosol selon la revendication 58, **caractérisé en ce que** le polymère fixant cationique est choisi parmi les homopolymères ou copolymères d'esters ou d'amides acryliques ou méthacryliques à fonctions aminées, les polysaccharides cationiques, les copolymères quaternaires de vinylpyrrolidone et de vinylimidazole, et les chitosanes.

60. Dispositif aérosol selon la revendication 58, **caractérisé en ce que** le polymère fixant anionique est choisi parmi les homopolymères ou copolymères d'acide acrylique et méthacrylique ou leurs sels, les copolymères d'acide crotonique, les copolymères d'acides ou d'anhydrides carboxyliques monoinsaturés en C₄-C₈, les polyacrylamides à groupements carboxylates, les homopolymères ou copolymères à groupes sulfoniques, les polyuréthanes anioniques, et les polymères siliconés greffés anioniques.

61. Dispositif aérosol selon la revendication 58, **caractérisé en ce que** le polymère fixant amphotère est choisi parmi les copolymères à motifs vinyliques acides et à motifs vinyliques basiques, les polyaminoamides réticulés et acylés, les polymères à motifs zwittérioniques, les polymères dérivés de chitosane, les copolymères alkyl(C₁-C₅)vinyléther/anhydride maléique modifiés, les polyuréthanes amphotères et les polymères siliconés greffés amphotères.

62. Dispositif aérosol selon la revendication 58, **caractérisé en ce que** le polymère fixant non ionique est choisi parmi les polyalkyloxazolines, les homopolymères et copolymères d'acétate de vinyle, les homopolymères et copolymères d'esters acryliques, les copolymères d'acrylonitrile, les homopolymères et copolymères de styrène, les polyamides, les homopolymères de vinyllactame différents des homopolymères de vinylpyrrolidone, les copolymères de vinyllactame, et les polymères siliconés greffés non ioniques.

63. Dispositif aérosol selon la revendication 58, **caractérisé en ce que** le polymère fixant est choisi parmi les polyuréthanes non ioniques, anioniques, cationiques ou amphotères.

64. Dispositif aérosol selon l'une des revendications précédentes tel que la concentration en polymère(s) fixant(s) additionnel(s) est comprise entre 0,1 et 20%, de préférence entre 0,5 et 10% en poids par rapport au poids total de la composition.

65. Dispositif aérosol selon l'une quelconque des revendications précédentes, **caractérisé par le fait qu'**elle contient un agent épaississant.

66. Dispositif aérosol selon la revendication 65, **caractérisé par** le fait l'agent épaississant est un polymère épaississant associatif.

67. Dispositif aérosol selon la revendication 65, **caractérisé par** le fait l'agent épaississant est un gélifiant.

68. Dispositif aérosol selon l'une des revendications 65 à 67, **caractérisée par le fait que** la concentration en agent épaississant est comprise entre 0,01 et 10%, de préférence entre 0,1 et 5% en poids par rapport au poids total de la composition.

69. Dispositif aérosol selon l'une quelconque des revendications précédentes, **caractérisée par le fait qu'**elle contient un tensioactif.

70. Dispositif aérosol selon la revendication 69, **caractérisé par le fait que** la concentration en tensioactif est comprise entre 0,01 et 40%, de préférence entre 0,1 et 30% en poids par rapport au poids total de la composition.

71. Dispositif aérosol selon l'une quelconque des revendications 1 à 70 tel qu'il est choisi parmi les dispositifs monocompartimentaux et les dispositifs bicompartimentaux.

72. Dispositif aérosol selon l'une quelconque des revendications 1 à 71 tel qu'il est choisi parmi les dispositifs monocompartimentaux et que le gaz propulseur est sélectionné parmi les gaz liquéfiés ou comprimés tels que l'azote, le diméthyl éther, les hydroxycarbures.

73. Dispositif aérosol selon la revendication 71 ou 72 tel que la concentration en agent(s) propulseur(s) représente de 6 à 70 %, de préférence 6 à 50 %, de façon plus préférée de 30 à 45 % du poids total de matières contenues dans ledit dispositif.

74. Procédé de traitement cosmétique pour la mise en forme ou le maintien de la coiffure, **caractérisé par** la vaporisation sur les cheveux du contenu d'un dispositif aérosol selon l'une quelconque des revendications 1 à 70.

75. Utilisation du contenu du dispositif aérosol selon l'une des revendications 1 à 70 pour la mise en forme ou le maintien des cheveux.

76. Utilisation du contenu du dispositif aérosol selon la revendication 75 pour obtenir une mise en forme des cheveux persistante dans le temps.

77. Utilisation du contenu du dispositif aérosol selon la revendication 75 pour obtenir une mise en forme des cheveux résistante à l'humidité.
